# EUROPEAN PATENT APPLICATION

(11) **EP 1 262 195 A1**
(43) Date of publication of application: **04.12.2002**
(21) Application number: 01908338.5
(22) Date of filing: 06.03.2001
(51) Int. Cl.: A61K 45/00, A61K 31/437, A61K 31/444, C07D 471/04, A61P 43/00, A61P 9/08, A61P 9/12, A61P 9/10, A61P 9/04

(54) **VASOACTIVE AGENTS**

(30) Priority: 07.03.2000 JP 2000067213
(71) Applicant: Takeda Chemical Industries, Ltd., Osaka-shi, Osaka 541-0045 (JP)
(72) Inventor: TARUI, Naoki, Nara-shi Nara 631-0061 (JP); SANTO, Takashi, Kobe-shi Hyogo 658-0015 (JP); MORI, Masaaki, Tsukuba-shi Ibaraki 305-0821 (JP); WATANABE, Hiroyuki, Kobe-shi Hyogo 651-2273 (JP)
(74) Representative: Lewin, John Harvey
(86) International application number: JP0101717
(87) International publication number: WO01066143

(57) **Abstract**

A vasoactive drug is provided which comprises a compound having GPR14-antagonizing activity or salts thereof.

Sequence Listing
<110> Takeda Chemical Industries, Ltd.
<120> Vasoactive agent
<130> 2698WOOP
<150> JP2000-067213
<151> 2000-03-07
<160> 4
<210> 1
<211> 37
<212> DNA
<213> Artificial Sequence
<220>
<223>
<400> 1
tcgtgagtcg accaccatgg cgctgacccc cgagtcc     37
<210> 2
<211> 33
<212> DNA
<213> Artificial Sequence
<220>
<223>
<400> 2
gcctggacta gtgccgcccc tccgcgtgct cac     33
<210> 3
<211> 1215
<212> DNA
<213> Human
<400> 3
tcgtgagtcg accaccatgg cgctgacccc cgagtccccg agcagcttcc ctgggctggc     60

cgccaccggc agctctgtgc cggagccgcc tggcggcccc aacgcaaccc tcaacagctc 120
ctgggccagc ccgaccgagc ccagctccct ggaggacctg gtggccacgg gcaccattgg 180
gactctgctg tcggccatgg gcgtggtggg cgtggtgggc aacgcctaca cgctggtggt 240
cacctgccgc tccctgcgtg cggtggcctc catgtacgtc tacgtggtca acctggcgct 300
ggccgacctg ctgtacctgc tcagcatccc cttcatcgtg gccacctacg tcaccaagga 360
gtggcacttc ggggacgtgg gctgccgcgt gctcttcggc ctggacttcc tgaccatgca 420
cgccagcatc ttcacgctga ccgtcatgag cagcgagcgc tacgctgcgg tgctgcggcc 480
gctggacacc gtgcagcgcc ccaagggcta ccgcaagctg ctggcgctgg gcacctggct 540
gctggcgctg ctgctgacgc tgcccgtgat gctggccatg cggctggtgc gccggggtcc 600
caagagcctg tgcctgcccg cctggggccc gcgcgcccac cgcgcctacc tgacgctgct 660
cttcgccacc agcatcgcgg ggcccgggct gctcatcggg ctgctctacg cgcgcctggc 720
ccgcgcctac cgccgctcgc agcgcgcctc cttcaagcgg gcccggcggc cgggggcgcg 780
cgcgctgcgc ctggtgctgg gcatcgtgct gctcttctgg gcctgcttcc tgcccttctg 840
gctgtggcag ctgctcgccc agtaccacca ggccccgctg gcgccgcgga cggcgcgcat 900
cgtcaactac ctgaccacct gcctcaccta cggcaacagc tgcgccaacc ccttcctcta 960
cacgctgctc accaggaact accgcgacca cctgcgcggc cgcgtgcggg gcccgggcag 1020
cgggggaggc cgggggcccg ttccctccct gcagccccgc gcccgcttcc agcgctgttc 1080
gggccgctcc ctgtcttcct gcagcccaca gcccactgac agcctcgtgc tggccccagc 1140
ggccccggcc cgacctgccc ccgagggtcc cagggccccg gcgtgagcac gcggaggggc 1200
ggcactagtc caggc 1215
<210> 4
<211> 389
<212> PRT
<213> Human
<400> 4
Met Ala Leu Thr Pro Glu Ser Pro Ser Ser Phe Pro Gly Leu Ala Ala
1               5                   10                  15
Thr Gly Ser Ser Val Pro Glu Pro Pro Gly Gly Pro Asn Ala Thr Leu
            20                  25                  30

Asn Ser Ser Trp Ala Ser Pro Thr Glu Pro Ser Ser Leu Glu Asp Leu
        35                  40                  45
Val Ala Thr Gly Thr Ile Gly Thr Leu Leu Ser Ala Met Gly Val Val
    50                  55                  60
Gly Val Val Gly Asn Ala Tyr Thr Leu Val Val Thr Cys Arg Ser Leu
65                  70                  75                  80
Arg Ala Val Ala Ser Met Tyr Val Tyr Val Val Asn Leu Ala Leu Ala
                85                  90                  95
Asp Leu Leu Tyr Leu Leu Ser Ile Pro Phe Ile Val Ala Thr Tyr Val
            100                 105                 110
Thr Lys Glu Trp His Phe Gly Asp Val Gly Cys Arg Val Leu Phe Gly
        115                 120                 125
Leu Asp Phe Leu Thr Met His Ala Ser Ile Phe Thr Leu Thr Val Met
    130                 135                 140
Ser Ser Glu Arg Tyr Ala Ala Val Leu Arg Pro Leu Asp Thr Val Gln
145                 150                 155                 160
Arg Pro Lys Gly Tyr Arg Lys Leu Leu Ala Leu Gly Thr Trp Leu Leu
                165                 170                 175
Ala Leu Leu Leu Thr Leu Pro Val Met Leu Ala Met Arg Leu Val Arg
            180                 185                 190
Arg Gly Pro Lys Ser Leu Cys Leu Pro Ala Trp Gly Pro Arg Ala His
        195                 200                 205
Arg Ala Tyr Leu Thr Leu Leu Phe Ala Thr Ser Ile Ala Gly Pro Gly
    210                 215                 220
Leu Leu Ile Gly Leu Leu Tyr Ala Arg Leu Ala Arg Ala Tyr Arg Arg
225                 230                 235                 240
Ser Gln Arg Ala Ser Phe Lys Arg Ala Arg Arg Pro Gly Ala Arg Ala
                245                 250                 255
Leu Arg Leu Val Leu Gly Ile Val Leu Leu Phe Trp Ala Cys Phe Leu

            260                 265                 270

Pro Phe Trp Leu Trp Gln Leu Leu Ala Gln Tyr His Gln Ala Pro Leu

        275                 280                 285

Ala Pro Arg Thr Ala Arg Ile Val Asn Tyr Leu Thr Thr Cys Leu Thr

    290                 295                 300

Tyr Gly Asn Ser Cys Ala Asn Pro Phe Leu Tyr Thr Leu Leu Thr Arg

305                 310                 315                 320

Asn Tyr Arg Asp His Leu Arg Gly Arg Val Arg Gly Pro Gly Ser Gly

                325                 330                 335

Gly Gly Arg Gly Pro Val Pro Ser Leu Gln Pro Arg Ala Arg Phe Gln

            340                 345                 350

Arg Cys Ser Gly Arg Ser Leu Ser Ser Cys Ser Pro Gln Pro Thr Asp

        355                 360                 365

Ser Leu Val Leu Ala Pro Ala Ala Pro Ala Arg Pro Ala Pro Glu Gly

    370                 375                 380

Pro Arg Ala Pro Ala

385

## Description

### Technical Field

The present invention relates to GPR14 antagonistic agents and vasoactive agents which comprise, as an active agent, a compound having GPR 14-antagonizing activity or salts thereof, and more particularly to a novel quinoline derivatives having GPR 14-antagonizing activity or salts thereof.

### Background of the Invention

Urotensin II was discovered as one of peptide hormones having a potent vasoconstrictive activity, and has been proved to have extremely higher vasoconstrictive activity than endothelin, which is the potentest vasoconstrictor among those which are currently known to have vasoconstrictive activity on mammal arteria. The receptor for urotensin II is GPR 14 protein, which is one of orphan receptors (see, Nature, vol. 401, p.282 (1999)), and antagonists of this receptor may possibly be used to treat ischemic myocardial infarction, congestive heart failure and the like. However, none of such antagonists have been reported yet.

An agent which exhibits GPR 14-antagonizing activity may probably be used as a therapeutic agent to treat ischemic myocardial infarction, congestive heart failure and the like, and thus can be expected for use as a novel vasoactive drug.

The present invention provides, based on such GPR 14-antagonizing activity, a vasoactive agent (particularly vasoconstriction inhibitor) which is useful for preventing and/or treating, for example, hypertension, arteriosclerosis, hypercardia, myocardial infarction and heart failure, as well as a novel quinoline derivative having GPR 14-antagonizing activity or salts thereof.

### Disclosure of the Invention

The present inventors developed the present invention, after intense studies examining compounds having GPR 14-antagonizing activity, based on the findings that a compound having the formula (I) below or salts thereof (hereinafter sometimes referred to as "compound (I)") can exhibit an excellent GPR 14-antagonizing activity.

In summary, the present invention relates to:
1) a vasoactive agent which comprises a compound having GPR 14-antagonizing activity or salts thereof;
2) the agent according to 1) above which is a vasoconstriction inhibitor;
3) the agent according to 1) above which is an agent for preventing and/or treating hypertension, arteriosclerosis, hypercardia, myocardial infarction or heart failure;
4) a GPR 14 antagonistic agent which comprises a non-peptide compound or salts thereof;
5) a GPR 14 antagonistic agent which comprises a quinoline derivative or salts thereof;
6) a GPR 14 antagonistic agent which comprises 4-amino quinoline derivative or salts thereof;
7) a GPR 14 antagonistic agent which comprises a compound having the formula (I): 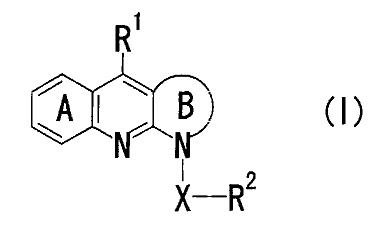 wherein A is a benzene ring which may be substituted, B is a 5- to 8-membered ring which may be substituted , X is a divalent group containing 1 to 4 atom(s) in its linear chain, R¹ is an amino group which may be substituted, and R² is a cyclic group which may be substituted; or salts thereof;
8) the agent according to 7) above, wherein A is a benzene ring which may be substituted by: (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂-₄ alkanoyl, (15') C₁₋₄ alkylsulfonyl or (16') C₁₋₄ alkylsulfinyl; (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl, (16') C₁₋₄ alkylsulfinyl; (3) a nitro group; (4) a halogen atom; (5) an amino group which may be substituted by (1') alkyl group which may be substituted, (2') cycloalkyl group which may be substituted, (3') alkenyl group which may be substituted, (4') cycloalkenyl group which may be substituted, (5') aralkyl group which may be substituted, (6') formyl or acyl group which may be substituted, (7') aryl group which may be substituted, (8') heterocyclic group which may be substituted; or (6) a group represented by the formula: R⁴-Y- (wherein Y is an oxygen atom or sulfur atom which may be oxidized, and R⁴ is (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl or (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl);
9) the agent according to 7) above, wherein B is a 5- to 8-membered saturated ring which may be substituted by (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10')C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (3) a nitro group; (4) a halogen atom; (5) an amino group which may be substituted by (1') alkyl group which may be substituted, (2') cycloalkyl group which may be substituted, (3') alkenyl group which may be substituted, (4') cycloalkenyl group which may be substituted, (5') aralkyl group which may be substituted, (6') formyl or acyl group which may be substituted, (7') aryl group which may be substituted or (8') heterocyclic group which may be substituted; (6) a group represented by the formula: R⁴-Y-(wherein Y is oxygen atom or sulfur atom which may be oxidized, and R⁴ is (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl, (16') C₁₋₄ alkylsulfinyl; or (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl); or (7) an oxo-group;
10) the agent according to 7) above, wherein a C₁₋₄ alkylene group which may be substituted by (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (3) a nitro group; (4) a halogen atom; (5) an amino group which may be substituted by (1') alkyl group which may be substituted, (2') cycloalkyl group which may be substituted, (3') alkenyl group which may be substituted, (4') cycloalkenyl group which may be substituted, (5') aralkyl group which may be substituted, (6') formyl or acyl group which may be substituted, (7') aryl group which may be substituted, (8') heterocyclic group which may be substituted; (6) a group represented by the formula: R⁴-Y-(wherein Y is oxygen atom or sulfur atom which may be oxidized, and R⁴ is (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; or (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl); or (7) an oxo-group;
11) the agent according to 7) above, wherein X is a methylene group;
12) the agent according to 7) above, wherein R¹ is an amino group which may be substituted by one or two selected from the group consisting of (1) halogen atom, (2) nitro, (3) cyano, (4) hydroxy group, (5) thiol group which may be substituted, (6) amino group which may be substituted, (7) carboxyl group which may be esterified or amidated, (8) C₁₋₄ alkyl which may be substituted by halogen atom or C₁₋₄ alkoxy, (9) C₁₋₄ alkoxy which may be substituted by halogen atom or C₁₋₄ alkoxy, (10) C₁₋₄ alkylenedioxy, (11) phenyl-C₁₋₄ alkyl, (12) C₃₋₇ cycloalkyl, (13) formyl, (14) C₂₋₄ alkanoyl, (15) C₁₋₄ alkylsulfonyl or (16) lower alkyl group which may be substituted by C₁₋₄ alkylsulfinyl;
13) the agent according to 7) above, wherein R² is a 5- or 6-membered cyclic group which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl;
14) the agent according to 7) above, wherein R² is a 5- or 6-membered aromatic ring group which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl;
15) the agent according to 7) above, wherein R² is a phenyl group which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl;
16) a GPR 14 antagonistic agent which comprises a compound of the formula (II): 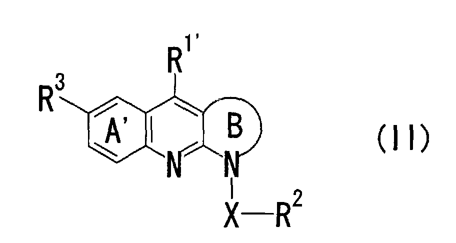 wherein A' is a benzene ring which may have one or more substituent(s) in addition to substituent R³, B is a 5- to 8-membered ring which may be substituted, X is a divalent group containing 1 to 4 atoms in its linear chain, R^{1'} is a substituted amino group, R² is a cyclic group which may be substituted, R³ is a hydrocarbon group which may be substituted, a heterocyclic group which may be substituted, a nitro group, a halogen atom, an amino group which may be substituted or a group represented by the formula: R⁴-Y-(wherein Y is an oxygen atom or sulfur atom which may be oxidized, and R⁴ is a hydrocarbon group which may be substituted or a heterocyclic group which may be substituted); or salt thereof;
17) a method for vasoactivating which comprises administering an effective amount of a compound having GPR 14-antagonizing activity or salts thereof;
18) use of a compound having GPR 14-antagonizing activity or salts thereof for manufacturing vasoactive agents;
19) a compound of the formula (II): 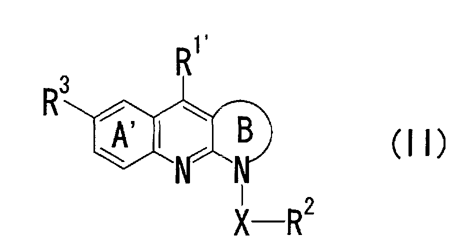 wherein A' is a benzene ring which may have one or more substituent(s) in addition to substituent R³, B is a 5- to 8-membered ring which may be substituted, X is a divalent group containing 1 to 4 atoms in its linear chain, R¹' is a substituted amino group, R² is a cyclic group which may be substituted, R³ is a hydrocarbon group which may be substituted, heterocyclic group which may be substituted, nitro group, halogen atom, amino group which may be substituted or a group represented by the formula: R⁴-Y-(wherein Y is an oxygen atom or sulfur atom which may be oxidized, and R⁴ is a hydrocarbon group which may be substituted or heterocyclic group which may be substituted), with the proviso that the compound is not 2-({1-[(benzyloxy)carbonyl]-6-methoxy-3-methyl-1H-pyrazolo[3,4-b]quinoline-4-yl}amino)acetic acid tert-butyl ester,2-({1-[(benzyloxy)carbonyl]-6-methoxy-3-methyl-1H-pyrazolo[3,4-b]quinoline-4-yl}amino)acetic acid, 2-({1-[(benzyloxy)carbonyl]-6-methoxy-3-methyl-1H-pyrazolo[3,4-b]quinoline-4-yl}amino)acetic acid sodium salt, 2-({1-[(benzyloxy)carbonyl]-6-methoxy-3-methyl-1H-pyrazolo[3,4-b]quinoline-4-yl}amino)acetyl-alanine tert-butyl ester,2-({1-[(benzyloxy)carbonyl]-6-methoxy-3-methyl-1H-pyrazolo[3,4-b]quinoline-4-yl}amino)acetylmethionine tert-butyl ester, and 2-({1-[(benzyloxy)carbonyl]-6-methoxy-3-methyl-1H-pyrazolo[3,4-b]quinoline-4-yl}amino)acetylleucine tert-butyl ester; or salts thereof;
20) a compound of the formula (II): 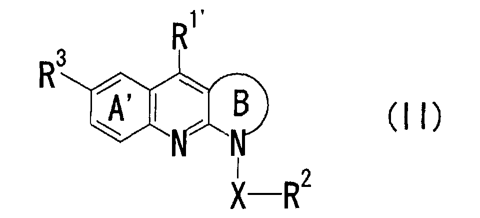 wherein A' is a benzene ring which may have one or more substituent(s) in addition to substituent R³, B is a 5- to 8-membered ring which may be substituted, X is C₁₋₄ alkylene group which may be substituted, R¹' is a substituted amino group, R² is a cyclic group which may be substituted, R³ is a hydrocarbon group which may be substituted, heterocyclic group which may be substituted, nitro group, halogen atom, amino group which may be substituted or a group represented by the formula: R⁴-Y- (wherein Y is an oxygen atom or sulfur atom which may be oxidized, and R⁴ is a hydrocarbon group which may be substituted or heterocyclic group which may be substituted; or salts thereof;
21) a prodrug of the compound or salts thereof according to 19) or 20) above;
22) the compound according to 19) or 20) above, wherein R³ is (1) a lower alkyl group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (2) a halogen group; (3) a phenyl group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; or (4) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl;
23) the compound according to 19) or 20) above, wherein R³ is (1) a lower alkyl group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; or (2) a halogen atom;
24) the compound according to 19) or 20) above, wherein B is a 5- to 8-membered saturated ring which may be substituted by: (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (3) a nitro group; (4) a halogen atom; (5) an amino group which may be substituted by (1') alkyl group which may be substituted, (2') cycloalkyl group which may be substituted, (3') alkenyl group which may be substituted, (4') cycloalkenyl group which may be substituted, (5') aralkyl group which may be substituted, (6') formyl or acyl group which may be substituted, (7') aryl group which may be substituted, (8') heterocyclic group which may be substituted; (6) a group represented by the formula: R⁴-Y- (wherein Y is an oxygen atom or sulfur atom which may be oxidized, and R⁴ is (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl, or (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl); or (7) an oxo-group;
25) the compound according to 19) or 20) above, wherein X is a methylene group;
26) the compound according to 19) or 20) above, wherein R² is a 5- or 6-membered cyclic ring which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl;
27) the compound according to 19) or 20) above, wherein R² is a 5- or 6-membered aromatic ring group which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl;
28) the compound according to 19) or 20) above, wherein R² is a phenyl group which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl;
29) the compound according to 19) or 20) above, wherein R^{1'} is an amino group which is substituted by a lower alkyl group which may be substituted by one or two of (1) halogen atom, (2) nitro, (3) cyano, (4) hydroxy group, (5) thiol group which may be substituted, (6) amino group which may be substituted, (7) carboxyl group which may be esterified or amidated, (8) C₁₋₄ alkyl which may be substituted by halogen atom or C₁₋₄ alkoxy, (9) C₁₋₄ alkoxy which may be substituted by halogen atom or C₁₋₄ alkoxy, (10) C₁₋₄ alkylenedioxy, (11) phenyl-C₁₋₄ alkyl, (12) C₃₋₇ cycloalkyl, (13) formyl, (14) C₂₋₄ alkanoyl, (15) C₁₋₄ alkylsulfonyl or (16) C₁₋₄ alkylsulfinyl;
30) a pharmaceutical composition which comprises a compound or salts thereof according to 19) or 20) above or prodrugs thereof;
31) the pharmaceutical composition according to 30) above wherein said pharmaceutical composition is a GPR 14 antagonistic agent;
32) a method for antagonizing GPR14 which comprises administering an effective amount of a compound of the formula (I): 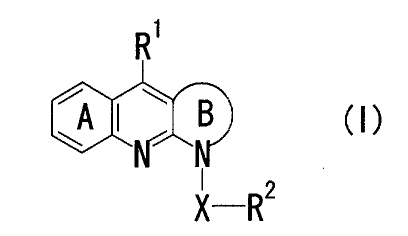 [wherein A is a benzene ring which may be substituted, B is a 5- to 8-membered ring which may be substituted, X is a divalent group containing 1 to 4 atom(s) in its linear chain, R¹ is an amino group which may be substituted, and R² is a cyclic group which may be substituted] or salts thereof;
33) a method for antagonizing GPR14 which comprises administering an effective amount of a compound of the formula (II) 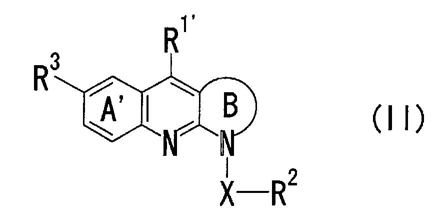 [wherein A' is a benzene ring which may have one or more substituent(s) in addition to substituent R³, B is a 5- to 8-membered ring which may be substituted, X is a divalent group containing 1 to 4 atoms in its linear chain, R¹' is a substituted amino group, R² is a cyclic group which may be substituted, R³ is a hydrocarbon group which may be substituted, heterocyclic group which may be substituted, nitro group, halogen atom, amino group which may be substituted or a group represented by the formula: R⁴-Y-(wherein Y is an oxygen atom or sulfur atom which may be oxidized, and R⁴ is a hydrocarbon group which may be substituted or heterocyclic group which may be substituted)] or salts thereof;
34) use of a compound of the formula (I): 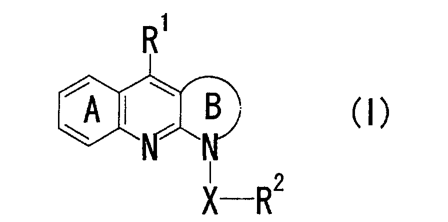 [wherein A is a benzene ring which may be substituted, B is a 5- to 8-membered ring which may be substituted, X is a divalent group containing 1 to 4 atom(s) in its linear chain, R¹ is an amino group which may be substituted, and R² is a cyclic group which may be substituted] or salts thereof for manufacturing a GPR 14 antagonistic agent; and
35) use of a compound of the formula (II): 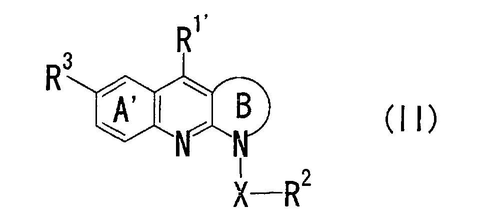 [wherein A' is a benzene ring which may have one or more substituent(s) in addition to substituent R³, B is a 5- to 8-membered ring which may be substituted, X is a divalent group containing 1 to 4 atoms in its linear chain, R¹' is a substituted amino group, R² is a cyclic group which may be substituted, R³ is a hydrocarbon group which may be substituted, heterocyclic group which may be substituted, nitro group, halogen atom, amino group which may be substituted or a group represented by the formula: R⁴-Y-(wherein Y is an oxygen atom or sulfur atom which may be oxidized, and R⁴ is a hydrocarbon group which may be substituted or heterocyclic group which may be substituted)] or salts thereof for manufacturing a GPR 14 antagonistic agent.

The term "GPR 14-antagonizing activity" used herein refers to an activity to competitively or non-competitively inhibit the binding of a ligand (e.g., urotensin II) to GPR14 protein on a cell membrane.

The present invention provides, based on such GPR 14-antagonizing activity, drugs which exhibit a variety of effects on blood vessels (e.g., accentuation or suppression of vasoconstriction). Among them, vasoconstriction inhibitors may preferably be used which reduce urotensin II-induced potent vasoconstriction. The vasoconstriction inhibitors can be used for preventing and/or treating a variety of diseases. Particularly, they may preferably be used for preventing and/or treating hypertension, arteriosclerosis, hypercardia, myocardial infarction, heart failure and the like, and more preferably, ischemic myocardial infarction, congestive heart failure and the like.

Preferable examples of compounds having GPR 14-antagonizing activity or salts thereof to be used in the present invention include non-peptide compounds having GPR 14-antagonizing activity or salts thereof which may advantageously exhibit a longer period of reaction time. Particularly, quinoline derivative may be preferable, and 4-aminoquinoline derivative may be more preferable.

Among them, preferable examples of compounds having GPR 14-antagonizing activity or salts thereof which can be used in the present invention include those having the formula (I) below: 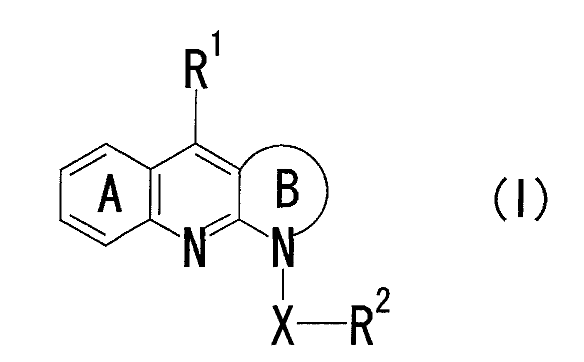 wherein A is a benzene ring which may be substituted, B is a 5- to 8-membered ring which may be substituted, X is a divalent group containing 1 to 4 atom(s) in its linear chain, R¹ is an amino group which may be substituted, and R² is a cyclic group which may be substituted; or salts thereof.

In the above-described formula, examples of substituent which the "benzene ring which may be substituted" (represented by A) may have include hydrocarbon group which may be substituted; heterocyclic group which may be substituted; nitro group; halogen atom; amino group which may be substituted; a group represented by the formula: R⁴-Y- (wherein Y is an oxygen atom or sulfur atom which may be oxidized, and R⁴ is a hydrocarbon group which may be substituted or heterocyclic group which may be substituted); cyano group; acyl group which may be substituted; carboxyl group which may be esterified or amidated; and the like.

Examples of hydrocarbon group in the "hydrocarbon group which may be substituted" which the "benzene ring which may be substituted" (represented by A) may have or which are represented by R⁴ include
(1) alkyl (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl or decyl, etc., and preferably lower (C₁₋₆) alkyl, etc.);
(2) cycloalkyl (e.g.,C₃₋₈ cycloalkyl such as cyclopropyl, cyclobutyl, cyclopentyl, cyclohexyl, cycloheptyl, etc.), wherein the cycloalkyl may be condensed with a benzene ring to form indan (e.g., indan 1-yl, indan 2-yl, etc.), tetrahydronaphthalene (e.g., tetrahydronaphthalene-5-yl, tetrahydronaphthalene-6-yl, etc.)(preferably indan, etc.), and wherein the cycloalkyl may be crosslinked with via a C₁₋₂ linear atomic chain to form a closslinked cyclic hydrocarbon group such as bicyclo [2.2.1] heptyl, bicyclo [2.2.2] octyl, bicyclo [3.2.1] octyl, bicyclo [3.2.2] nonyl, etc. (preferably cyclohexyl having a closslinkage via a C₁₋₂ linear atomic chain, etc., and more preferably bicyclo [2.2.1] heptyl etc.);
(3) alkenyl (e.g., C₂₋₁₀ alkenyl such as vinyl, allyl, crotyl, 2-pentenyl or 3-hexenyl, etc., preferably lower (C₂₋₆) alkenyl);
(4) cycloalkenyl (e.g., C₃₋₈ cycloalkenyl such as 2-cyclopentenyl, 2-cyclohexenyl, 2-cyclopentenylmethyl, 2-cyclohexenylmethyl, etc.);
(5) alkynyl (e.g., C₂₋₁₀ alkynyl such as ethynyl, 1-propynyl, 2-propynyl, 1-butynyl, 2-pentynyl, 3-hexynyl, etc., preferably lower (C₂₋₆) alkynyl, etc.);
(6) aryl (e.g., C₆₋₁₄ aryl such as phenyl or naphthyl, etc., preferably C₆₋₁₀ aryl, and more preferably phenyl, etc.);
(7) aralkyl (e.g., C₁₋₆ alkyl having 1 to 3 C₆₋₁₄ allyl groups, preferably phenyl-C₁₋₄ alkyl (e.g., benzyl, phenethyl, etc.).

Particularly, alkyl may be preferable, C₁₋₄ alkyl (e.g., methyl, ethyl, etc.) may be more preferable, and methyl may be most preferable.

The hydrocarbon group may have one or more substituent(s), and preferably 1 to 3 substituent(s). Substituents include, for example, halogen (e.g., fluorine, chlorine, bromine, iodine, etc.), nitro, cyano, hydroxy group, thiol group which may be substituted (e.g., thiol, C₁₋₄ alkylthio, etc.), amino group which may be substituted (e.g., amino, mono-C₁₋₄ alkylamino, di-C₁₋₄ alkylamino, mono-C₂₋₅ alkanoylamino, or 5- or 6-membered cyclic amino such as tetrahydropyrrole, piperazine, piperidine, morpholine, thiomorpholine, pyrrole, imidazole, etc.), phenyl-lower (C₁₋₄) alkyl, C₃₋₇ cycloalkyl, carboxyl group which may be esterified or amidated (e.g., carboxyl, C₁₋₄ alkoxy-carbonyl, lower (C₇₋₁₀) aralkyloxy-carbonyl, carbamoyl, mono-C₁₋₄ alkylcarbamoyl, di-C₁₋₄ alkylcarbamoyl, etc.), C₁₋₄ alkyl which may be substituted by halogen atom or C₁₋₄ alkoxy (e.g., trifluoromethyl, methyl, ethyl, etc.), C₁₋₄ alkoxy which may be substituted by halogen atom or C₁₋₄ alkoxy (e.g., methoxy, ethoxy, trifluoromethoxy, trifluoroethoxy, etc.), C₁₋₄ alkylenedioxy (e.g., -O-CH₂-O-, -O-CH₂-CH₂-O-, etc.), formyl, C₂₋₄ alkanoyl (e.g., acetyl, propionyl, etc.), C₁₋₄ alkylsulfonyl (e.g., methanesulfonyl, ethanesulfonyl, etc.), C₁₋₄ alkylsulfinyl (e.g., methanesulfinyl, ethanesulfinyl, etc.), etc.

Examples of heterocyclic group in the "heterocyclic group which may be substituted" which the "benzene ring which may be substituted" (represented by A) may have, and in the "heterocyclic group which may be substituted" (represented by R⁴), include those which may be formed by eliminating one hydrogen atom from a 5- to 8-membered aromatic heterocyclic ring and saturated or unsaturated non-aromatic (aliphatic) heterocyclic ring containing at least 1 (preferably 1 to 4, and more preferably 1 or 2) of 1 to 3 (preferably 1 or 2) heteroatom(s) selected from the group consisting of oxygen, sulfur and nitrogen atoms.

Examples of "aromatic heterocyclic ring" include 5-to 8-membered (preferably 5- or 6-membered) aromatic monocyclic heterocyclic rings (e.g., furan, thiophene, pyrrole, oxazole, isoxazole, thiazole, isothiazole, imidazole, pyrazole, 1,2,3-oxadiazole, 1,2,4-oxadiazole, 1,3,4-thiadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine or triazine). Examples of "non-aromatic heterocyclic ring" include 5- to 8-membered (preferably 5- or 6-membered) saturated or unsaturated monocyclic non-aromatic heterocyclic ring (aliphatic heterocyclic ring) such as pyrrolidine, tetrahydrofuran, tetrahydrothiophene, thiolane, dithiolane, oxathiolane, pyrroline, imidazolidine, imidazoline, pyrazolidine, pyrazoline, oxazine, oxadiazine, thiazine, thiadiazine, piperidine, morpholine, thiomorpholine, tetrahydropyran, piperazine, pyran, oxepin, thiepin or azepine; and 5- to 8-membered non-aromatic heterocyclic rings comprising any of the above-described aromatic monocyclic heterocyclic ring with all or portion of double bonds therein being saturated.

Examples of "heterocyclic group" in the "heterocyclic group which may be substituted" which "benzene ring which may be substituted" represented by A may have, or in the "heterocyclic group which may be substituted " represented by R⁴, include those formed by eliminating one hydrogen atom from a condensed ring formed by condensation of two or three (preferably two) rings selected from the group consisting of the above-listed monocyclic heterocyclic rings (monocyclic aromatic heterocyclic rings and monocyclic non-aromatic heterocyclic rings) and 5- to 8-membered cyclic hydrocarbons (e.g., 5- to 8-membered (preferably 5- or 6-membered) saturated or unsaturated alicyclic hydrocarbons such as C₅₋₈ cycloalkane, C₅₋₈ cycloalkene, C₅₋₈ cycloalkadiene, etc., and 6-membered aromatic hydrocarbon such as benzene). Those condensed rings may be saturated, partially unsaturated or aromatic.

Preferable examples of such condensed ring include those comprising two identical or different heterocyclic rings (preferably one is a heterocyclic ring and the other an aromatic heterocyclic ring, and more preferably two identical or different aromatic heterocyclic rings), and those comprising one heterocyclic ring and one homocyclic ring (preferably one is a heterocyclic ring and the other a benzene ring, and more preferably one is an aromatic heterocyclic ring and the other a benzene ring). Particular examples of such condensed ring include indole, benzothiophene, benzofuran, benzimidazole, imidazo[1,2-a]pyridine, quinoline, isoquinoline, cinnoline, etc.

The "heterocyclic group which may be substituted" which the "benzene ring which may be substituted" represented by A may have, or which is represented by R⁴, may have one or more substituent(s), including, for example, those similar to the above-listed substituents which the "hydrocarbon group which may be substituted" that the "benzene ring which may be substituted" represented by A may have may have.

Examples of the "halogen atom" which the "benzene ring which may be substituted" represented by A may have include fluorine, chlorine, bromine and iodine.

Examples of the "amino group which may be substituted" which the "benzene ring which may be substituted" represented by A may have include those similar to the "amino group which may be substituted" represented by R¹ (described later). Among them, amino groups are preferable which may have one or two substituents selected from the group consisting of "hydrocarbon group which may be substituted" (those similar to the above-listed "hydrocarbon group which may be substituted" which the "benzene ring which may be substituted" represented by A may have); "heterocyclic group which may be substituted" (those similar to the above-listed "heterocyclic group which may be substituted" which "benzene ring which may be substituted" represented by A may have); and "acyl group which may be substituted" (those similar to the "acyl group which may be substituted" which "benzene ring which may be substituted" represented by A may have, described later). Particularly preferable are, amino groups which may have one or two alkyl groups which may be substituted [e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, which may have 1 to 3 substituent(s) selected from the group consisting of halogen (e.g., fluorine, chlorine, bromine, iodine, etc.), nitro, cyano, hydroxy group, thiol group which may be substituted (e.g., thiol or C₁₋₄ alkylthio), amino group which may be substituted (e.g., amino, mono-C₁₋₄ alkylamino, di-C₁₋₄ alkylamino, 5- or 6-membered cyclic amino such as tetrahydropyrrole, piperazine, piperidine, morpholine, thiomorpholine, pyrrole, imidazole, etc.), phenyl lower (C₁₋₄) alkyl, C₃₋₇ cycloalkyl, carboxyl group which may be esterified or amidated (e.g., carboxyl, C₁₋₄ alkoxycarbonyl, lower (C₇₋₁₀) aralkyloxy-carbonyl, carbamoyl, mono-C₁₋₄ alkylcarbamoyl, di-C₁₋₄ alkylcarbamoyl, etc.), C₁₋₄ alkyl which may be substituted by halogen atom or C₁₋₄ alkoxy (e.g., trifluoromethyl, methyl, ethyl, etc.), C₁₋₄ alkoxy which may be substituted by halogen atom or C₁₋₄ alkoxy (e.g., methoxy, ethoxy, trifluoromethoxy, trifluoroethoxy, etc.), C₁₋₄ alkylenedioxy (e.g.,-O-CH₂-O-, -O-CH₂-CH₂-O-, etc.), formyl, C₂₋₄ alkanoyl (e.g., acetyl, propionyl, etc.), C₁₋₄ alkylsulfonyl (e.g., methanesulfonyl, ethanesulfonyl, etc.), C₁₋₄ alkylsulfinyl (e.g., methanesulfinyl, ethanesulfinyl, etc.)].

The "amino group which may be substituted" which the "benzene ring which may be substituted" represented by A may be an amino group in which the substituents of the amino group are bonded to each other to form a cyclic amino group (e.g., a cyclic amino group formed by eliminating one hydrogen atom from constituent nitrogen atom of a 5- or 6-membered ring with a binding arm on its nitrogen atom, such as tetrahydropyrrole, piperazine, piperidine, morpholine, thiomorpholine, pyrrole, imidazole, etc.). The cyclic amino group may have one or more (preferably 1 to 3) substituent(s), including halogen (e.g., fluorine, chlorine, bromine, iodine, etc.), nitro, cyano, hydroxy group, thiol group, amino group, carboxyl group, C₁₋₄ alkyl which may be halogenated (e.g., trifluoromethyl, methyl, ethyl, etc.), C₁₋₄ alkoxy which may be halogenated (e.g., methoxy, ethoxy, propoxy, butoxy, trifluoromethoxy, trifluoroethoxy, etc.), formyl, C₂₋₄ alkanoyl (e.g., acetyl, propionyl, etc.) or C₁₋₄ alkylsulfonyl (e.g., methanesulfonyl, ethanesulfonyl, etc.).

The "acyl group which may be substituted" which the "benzene ring which may be substituted" represented by A may have include those comprising carbonyl group or sulfonyl group bonded with, for example: hydrogen; "hydrocarbon group which may be substituted" (e.g., those similar to the above-listed "hydrocarbon group which may be substituted" which the "benzene ring which may be substituted" represented by A may have); or "heterocyclic group which may be substituted" (e.g., those similar to the above-listed "heterocyclic group which may be substituted" which the "benzene ring which may be substituted" represented by A may have). Preferable examples are those comprising carbonyl group or sulfonyl group bonded with, for example:
(1) hydrogen;
(2) alkyl which may be substituted (e.g.,C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl);
(3) cycloalkyl which may be substituted (e.g., C₃₋₇ cycloalkyl such as cyclopropyl, cyclobutyl, cyclopentyl, cyclohexyl or cycloheptyl, etc.);
(4) alkenyl which may be substituted (e.g., C₂₋₁₀ alkenyl such as allyl, crotyl, 2-pentenyl, 3-hexenyl, etc., preferably lower (C₂₋₆) alkenyl);
(5) cycloalkenyl which may be substituted (e.g., C₃₋₇ cycloalkenyl such as 2-cyclopentenyl, 2-cyclohexenyl, 2-cyclopentenylmethyl, 2-cyclohexenylmethyl, etc.); and
(6) 5- or 6-membered monocyclic aromatic group which may be substituted (e.g., phenyl, pyridyl, etc.), including acetyl, propionyl, butyryl, isobutyryl, valeryl, isovaleryl, pivaloyl, hexanoyl, heptanoyl, octanoyl, cyclobutane carbonyl, cyclopentane carbonyl, cyclohexane carbonyl, cycloheptane carbonyl, crotonyl, 2-cyclohexene carbonyl, benzoyl, nicotinoyl, methanesulfonyl, ethanesulfonyl, etc. The above-described (2) alkyl which may be substituted, (3) cycloalkyl which may be substituted, (4) alkenyl which may be substituted, (5) cycloalkenyl which may be substituted, and (6) 5- or 6-membered monocyclic aromatic group which may be substituted may preferably have 1 to 3 substituent(s), including halogen (e.g., fluorine, chlorine, bromine or iodine); nitro; cyano; hydroxy group; thiol group which may be substituted (e.g., thiol, C₁₋₄ alkylthio, etc.); amino group which may be substituted (e.g., amino, mono-C₁₋₄ alkylamino, di-C₁₋₄ alkylamino, 5- or 6-membered cyclic amino such as tetrahydropyrrole, piperazine, piperidine, morpholine, thiomorpholine, pyrrole, imidazole, etc.); carboxyl group which may be esterified or amidated (e.g., carboxyl, C₁₋₄ alkoxycarbonyl, carbamoyl, mono-C₁₋₄ alkylcarbamoyl, di-C₁₋₄ alkylcarbamoyl, etc.); C₁₋₄ alkyl which may be substituted by halogen atom or C₁₋₄ alkoxy (e.g., trifluoromethyl, methyl, ethyl, etc.); C₁₋₄ alkoxy which may be substituted by halogen atom or C₁₋₄ alkoxy (e.g., methoxy, ethoxy, trifluoromethoxy, trifluoroethoxy, etc.); formyl; C₂₋₄ alkanoyl (e.g., acetyl, propionyl, etc.); C₁₋₄ alkylsulfonyl (e.g., methanesulfonyl, ethanesulfonyl, etc.); and C₁₋₄ alkylsulfinyl (e.g., methanesulfinyl, ethanesulfinyl, etc.), etc.

The "carboxyl group which may be esterified " which the "benzene ring which may be substituted" represented by A may have may include those comprising carbonyloxy group bonded with, for example: hydrogen; or "hydrocarbon group which may be substituted" (such as those similar to the above-listed "hydrocarbon group which may be substituted" which the "benzene ring which may be substituted" represented by A may have). Preferable examples are those comprising carbonyloxy group bonded with, for example:
(1) hydrogen;
(2) alkyl which may be substituted (e.g.,C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl);
(3) cycloalkyl which may be substituted (e.g., C₃₋₇ cycloalkyl such as cyclopropyl, cyclobutyl, cyclopentyl, cyclohexyl, cycloheptyl, etc.);
(4) alkenyl which may be substituted (e.g., C₂₋₁₀ alkenyl such as allyl, crotyl, 2-pentenyl, 3-hexenyl, etc., and preferably lower (C₂₋₆) alkenyl);
(5) cycloalkenyl which may be substituted (e.g., C₃₋₇ cycloalkenyl such as 2-cyclopentenyl, 2-cyclohexenyl, 2-cyclopentenylmethyl, 2-cyclohexenylmethyl, etc.); and
(6) aryl which may be substituted (e.g., phenyl, naphthyl, etc.)

More preferable are carboxyl, lower (C₁₋₆) alkoxycarbonyl and allyloxycarbonyl (e.g., methoxycarbonyl, ethoxycarbonyl, propoxycarbonyl, phenoxycarbonyl, naphthoxycarbonyl, etc.). The above-listed (2) alkyl which may be substituted, (3) cycloalkyl which may be substituted, (4) alkenyl which may be substituted, (5) cycloalkenyl which may be substituted, and (6) aryl which may be substituted may preferably have 1 to 3 substituent(s), including, for example: halogen (e.g., fluorine, chlorine, bromine, iodine, etc.); nitro; cyano; hydroxy group; thiol group which may be substituted (e.g., thiol, C₁₋₄ alkylthio, etc.); amino group which may be substituted (e.g., amino, mono-C₁₋₄ alkylamino, di-C₁₋₄ alkylamino, 5- or 6-membered cyclic amino such as tetrahydropyrrole, piperazine, piperidine, morpholine, thiomorpholine, pyrrole, imidazole, etc.); carboxyl group which may be esterified or amidated (e.g., carboxyl, C₁₋₄ alkoxycarbonyl, carbamoyl, mono-C₁₋₄ alkylcarbamoyl, di-C₁₋₄ alkylcarbamoyl, etc.); C₁₋₄ alkyl which may be substituted by halogen atom or C₁₋₄ alkoxy (e.g., trifluoromethyl, methyl, ethyl, etc.); C₁₋₄ alkoxy which may be substituted by halogen atom or C₁₋₄ alkoxy (e.g., methoxy, ethoxy, trifluoromethoxy, trifluoroethoxy, etc.); formyl; C₂₋₄ alkancyl (e.g., acetyl, propionyl, etc.); C₁₋₄ alkylsulfonyl (e.g., methanesulfonyl, ethanesulfonyl, etc.); and C₁₋₄ alkylsulfinyl (e.g., methanesulfinyl, ethanesulfinyl, etc.).

Examples of the "carboxyl group which may be amidated" which the "benzene ring which may be substituted" represented by A may have include those comprising a carbonyl group bonded with, for example:
(1) hydroxy group; or
(2) "amino group which may be substituted" (e.g., those similar to the above-listed "amino group which may be substituted" which the "benzene ring which may be substituted" represented by A may have).

The "benzene ring which may be substituted" represented by A may have 1 to 4 identical or different substituent(s) (preferably 1 or 2 substituent(s)) at any position(s) of the ring. When the "benzene ring which may be substituted" represented by A have two or more substituents, any two of the substituents may be bonded to each other to form, for example, lower (C₁₋₆) alkylene (e.g., trimethylene, tetramethylene, etc.), lower (C₁₋₆) alkyleneoxy (e.g., -CH₂-O-CH₂-, -O-CH₂-CH₂-, etc.), lower (C₁₋₆) alkylenedioxy (e.g., -O-CH₂-O-, -O-CH₂-CH₂-O-, etc.), lower (C₂₋₆) alkenylene (e.g.,-CH₂-CH=CH-, -CH₂-CH₂-CH=CH-, - CH₂-CH=CH-CH₂-, etc.), lower (C₄₋₆) alkadienylene (e.g., - CH=CH-CH=CH-, etc.).

Preferable examples of substituent which the "benzene ring which may be substituted" represented by A may have include: hydrocarbon group which may be substituted; heterocyclic group which may be substituted; nitro group; halogen atom; amino group which may be substituted; and a group represented by the formula: R⁴-Y- (wherein Y is oxygen atom or sulfur atom which may be oxidized and R⁴ is a hydrocarbon group which may be substituted or heterocyclic group which may be substituted). More preferable are hydrocarbon group which may be substituted, heterocyclic group which may be substituted, halogen atom, amino group which may be substituted and a group represented by the formula R⁴-Y- (wherein Y is oxygen atom or sulfur atom which may be oxidized and R⁴ is a hydrocarbon group which may be substituted or heterocyclic group which may be substituted), etc. Most preferable are lower (C₁₋₄) alkyl and halogen atom, etc.

Examples of "benzene ring which may be substituted" represented by A preferably include those having the formula below: 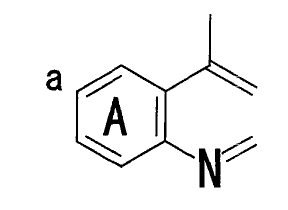 wherein the benzene ring has at least one substituent at position "a", and more preferably those having the formula below: 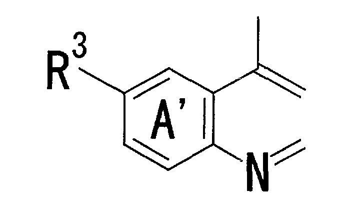 [wherein A' is a benzene ring which may have one or more substituent(s) in addition to substituent R³, R³ is hydrocarbon group which may be substituted, heterocyclic group which may be substituted, nitro group, halogen atom, amino group which may be substituted, or a group represented by the formula: R⁴-Y- (wherein Y is an oxygen atom or a sulfur atom which may be oxidized and R⁴ is a hydrocarbon group which may be substituted or a heterocyclic group which may be substituted)], and most preferably those having the formula below: 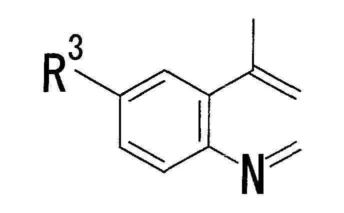 [wherein R³ is as described above]. In these formulae, examples of R³ preferably include hydrocarbon group which may be substituted, heterocyclic group which may be substituted, halogen atom, amino group which may be substituted, or a group represented by the formula: R⁴-Y-(wherein Y is an oxygen atom or a sulfur atom which may be oxidized and R⁴ is a hydrocarbon group which may be substituted or a heterocyclic group which may be substituted), more preferably hydrocarbon group which may be substituted, heterocyclic group which may be substituted or halogen atom, and most preferably lower alkyl group which may be substituted or halogen atom.

In the above-described formulae, examples of "a 5- to 8-membered ring which may be substituted" represented by B include saturated 5- to 8-membered ring which may have a substituent or substituents at any position(s) which can be substituted, having the formula below: 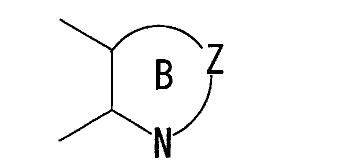 [wherein Z is a saturated divalent group with which ring B can form a saturated 5- to 8-membered ring which may be substituted]. Such saturated 5- to 8-membered rings may partially have unsaturated bond(s) and may form an aromatic ring. Ring B may preferably be a saturated 5- to 8-membered ring which may be substituted.

The term "saturated 5- to 8-membered ring" in "saturated 5- to 8-membered ring which may be substituted" represented by B refers to "a 5- to 8-membered ring in which all the bonds (except for the double bond via which the ring B forms, together with a quinoline ring, a condensed ring) which constitute the ring B are single bonds". The term "unsaturated 5- to 8-membered ring" in "unsaturated 5- to 8-membered ring which may be substituted" represented by B refers to "a 5- to 8-membered ring in which at least one of the bonds (except for the double bond via which the ring B forms, together with a quinoline ring, a condensed ring) which constitute the ring B is an unsaturated bond".

In the above-described formula, the saturated divalent group represented by Z may include any saturated groups provided that ring B can form, together with Z, a saturated 5- to 8-membered ring which may be substituted. In other words, Z may include any saturated divalent groups which have 2 to 5 atoms in its linear chain (preferably saturated divalent hydrocarbon group having 2 to 5 atoms in its linear chain). Particular examples are:
(1) - (CH₂)ₐ₁- (a1 indicates any integer 2 to 5);
(2) -(CH₂)_{b1}-Z¹-(CH₂)_{b2}- (b1 and b2 may indicate the same or different integers 0 to 4 provided that the sum of b1 and b2 is 1 to 4, and Z¹ is NH, O, S, SO or SO₂) ;
(3) -(CH₂)_{d1}-Z¹-(CH₂)_{d2}-Z²-(CH₂)_{d3}- (d1, d2 and d3 independently represent the same or different integers 0 to 3 provided that the sum of d1, d2 and d3 is 0 to 3, and Z¹ and Z² independently represent NH, O, S, SO or SO₂) ;
(4) -(CH₂)ₑ₁-Z¹-(CH₂)ₑ₂-Z²-(CH₂)ₑ₃-Z³-(CH₂)ₑ₄- (e1, e2, e3 and e4 independently represent the same or different integers 0 to 2 provided that the sum of d1, d2 and d3 is 0 to 2, and Z¹, Z² and Z³ independently represent NH, O, S, SO or SO₂), [preferably -(CH₂)ₐ₁- (a1 represents any integer 2 to 5)]. Particular examples are divalent groups, including -O-(CH₂) ₖ₁- (k1 indicates any integer 1 to 4), -(CH₂)ₖ₁-O- (k1 indicates any integer 1 to 4), -S-(CH₂)ₖ₁-(k1 indicates any integer 1 to 4), -(CH₂)ₖ₁-S- (k1 indicates any integer 1 to 4), -NH-(CH₂)ₖ₁- (k1 indicates any integer 1 to 4), -(CH₂)ₖ₁ -NH- (k1 indicates any integer 1 to 4), -(CH₂)ₖ₂- (k2 indicates any integer 2 to 5), -NH-NH-, -CH₂-NH-NH-, -NH-NH-CH₂- and -NH-CH₂-NH-, etc.

In addition to those "saturated 5- to 8-membered rings which may be substituted" listed above, examples of the "5- to 8-membered ring which may be substituted" represented by B in the above-described formula may also include a "saturated 5- to 8-membered ring which may be substituted" which partially has unsaturated bond(s) and a "5- to 8-membered aromatic ring which may be substituted". In this case, Z in the following formula: 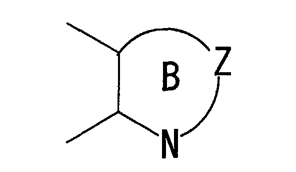 may represent a divalent group which is similar to any of the above-exemplified "saturated divalent group having 2 to 5 atoms in its linear chain" except for that the bonds in the "saturated divalent group having 2 to 5 atoms in its linear chain" have partially been converted into unsaturated bond(s).

Alternatively, the divalent group may have any one or more substituent(s) provided the substituent or substituents are capable of binding to the divalent group, including, for example, oxo-group and those groups similar to the above-listed "substituent" which the "benzene ring which may be substituted" represented by A may have. The divalent group may be substituted by 1 to 4 (preferably 1 or 2) identical or different substituent(s) at any position(s) thereof. When the divalent group has two or more substituents, two of them may bind to each other to form, for example, a lower (C₁₋₆) alkylene (e.g., trimethylene, tetramethylene, etc.), lower (C₁₋₆) alkyleneoxy (e.g., -CH₂-O-CH₂-, -O-CH₂-CH₂-, etc.), lower (C₁₋₆) alkylenedioxy (e.g., -O-CH₂-O-, -O-CH₂-CH₂-O-, etc.), lower (C₂₋₆) alkenylene (e.g., -CH₂-CH=CH-, -CH₂-CH₂-CH=CH-, -CH₂-CH=CH-CH₂-, etc.) or lower (C₄₋₆) alkadienylene (e.g., - CH=CH-CH=CH-, etc.), etc.

In the above-described formula, the "divalent group having 1 to 4 atom(s) in its linear chain" represented by X may include saturated divalent groups including:
(1)-(CH₂)_{f1}- (f1 indicates any integer 1 to 4);
(2) -(CH₂)_{g1}-X¹-(CH₂)_{g2}- (g1 and g2 independently indicate the same or different integers 0 to 3, provided that the sum of g1 and g2 is 1 to 3. X¹ is NH, O, S, SO or SO₂) ; and
(3) -(CH₂)ₕ₁-X¹-(CH₂)ₕ₂-X²-(CH₂)ₕ₃- (h1, h2 and h3 independently indicate the same or different integers 0 to 2 provided that the sum of h1, h2 and h3 is 0 to 2, and X¹ and X² independently represent NH, O, S, SO or SO₂, and at least one of X¹ and X² preferably indicates NH when h2 is 0), and those divalent groups of which bonds have partially been unsaturated.

Particular examples of such divalent group are -O-(CH₂)ₖ₃-(k3 is any integer 1 to 3), -(CH₂)ₖ₃-O- (k3 is any integer 1 to 3), -S-(CH₂)ₖ₃- (k3 is any integer 1 to 3), -(CH₂)ₖ₃-S-(k3 is any integer 1 to 3), -NH-(CH₂)ₖ₃- (k3 is any integer 1 to 3), -(CH₂)ₖ₃-NH- (k3 is any integer 1 to 3), -(CH₂)ₖ₄-(k4 is any integer 1 to 4), -CH=CH-, -C≡C-, -CO-NH-, SO₂-NH-, etc.

X may preferably indicate any divalent group except for -CO-O-CH₂-, more preferably divalent group having 1 to 4 carbon atom(s) which constitute its linear chain, and most preferably C₁₋₄ alkylene, C₂₋₄ alkenylene, particularly C₁₋₄ alkylene, especially methylene.

The divalent group represented by X may have one or more substituent(s) at any position or positions (preferably on carbon atom(s)), provided that the substituent or substituents are capable of binding to the divalent chain which constitutes the linear chain, including, for example, oxo-group and those similar to the above-listed substituent which the "benzene ring which may be substituted" represented by A may have. The divalent groups may be substituted by 1 to 4 (preferably 1 or 2) identical or different substituent(s) at any position or positions thereon.

Preferable examples of the substituent which the divalent groups represented by X may have include: lower (C₁₋₆) alkyl (e.g., methyl, ethyl, propyl, isopropyl, butyl, isobutyl, sec-butyl, tert-butyl, pentyl, isopentyl, neopentyl, hexyl, etc.); lower (C₃₋₇) cycloalkyl (e.g., cyclopropyl, cyclobutyl, cyclopentyl, cyclohexyl, cycloheptyl, etc.); formyl; lower (C₂₋₇) alkanoyl (e.g., acetyl, propionyl butyryl, etc.); lower (C₂₋₇) lower alkoxy-carbonyl; lower (C₁₋₆); lower alkoxy; hydroxy group; and oxo-group.

Examples of "amino group which may be substituted" represented by R¹ in the above-described formula include any amino group which may have one or two selected from the group consisting of: "hydrocarbon group which may be substituted" (e.g., those similar to the above-described "hydrocarbon group which may be substituted" which the "benzene ring which may be substituted" represented by A may have), "heterocyclic group which may be substituted" (e.g., those similar to the above-described "heterocyclic group which may be substituted" which the "benzene ring which may be substituted" represented by A may have) and "acyl group which may be substituted" (e.g., those similar to the above-described "acyl group which may be substituted" which the " benzene ring which may be substituted" represented by A may have). In the "amino group which may be substituted" represented by R¹, the substituents of the amino group may be bonded to each other to form a cyclic amino group (e.g., a cyclic amino group formed by eliminating one hydrogen atom from a constituent nitrogen atom of 5- or 6-membered ring such as tetrahydropyrrole, piperazine, piperidine, morpholine, thiomorpholine, pyrrole or imidazole, having a binding arm on the nitrogen atom). The cyclic amino group may have one or more substituent(s), and preferably 1 to 3 substituent(s), including halogen (e.g., fluorine, chlorine, bromine, iodine, etc.), nitro, cyano, hydroxy group, thiol group, amino group, carboxyl group, C₁₋₄ alkyl which may be halogenated (e.g., trifluoromethyl, methyl, ethyl, etc.), C₁₋₄ alkoxy which may be halogenated (e.g., methoxy, ethoxy, propoxy, butoxy, trifluoromethoxy, trifluoroethoxy, etc.), formyl, C₂₋₄ alkanoyl (e.g., acetyl, propionyl, etc.), C₁₋₄ alkylsulfonyl (e.g., methanesulfonyl, ethanesulfonyl, etc.), etc.

Examples of substituent which the "amino group which may be substituted" represented by R¹ may have preferably include
(1) alkyl which may be substituted (e.g., C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl, etc.);
(2) cycloalkyl which may be substituted (e.g., C₃₋₈ cycloalkyl such as cyclopropyl, cyclobutyl, cyclopentyl, cyclohexyl, cycloheptyl, cyano-octyl, etc.), wherein the cycloalkyl may be condensed with a benzene ring to form indan (e.g., indan-1-yl, indan-2-yl, etc.), tetrahydronaphthalene (e.g., tetrahydronaphthalene-5-yl, tetrahydronaphthalene-6-yl, etc.) etc. (preferably indan etc.), and wherein the cycloalkyl may be crosslinked via a C₁₋₂ linear atomic chain to form a crosslinked cyclic hydrocarbon group such as bicyclo [2.2.1] heptyl, bicyclo [2.2.2]octyl, bicyclo [3.2.1] octyl, bicyclo [3.2.2] nonyl etc. (preferably, cyclohexyl being crosslinked via a C₁₋₂ linear atomic chain, etc., and more preferably bicyclo [2.2.1] heptyl, etc.);
(3) alkenyl which may be substituted (e.g., C₂₋₁₀ alkenyl such as allyl, crotyl, 2-pentenyl, 3-hexenyl, etc., and preferably lower (C₂₋₆) alkenyl);
(4) cycloalkenyl which may be substituted (e.g., C₃₋₇ cycloalkenyl such as 2-cyclopentenyl, 2-cyclohexenyl, 2-cyclopentenylmethyl, 2-cyclohexenylmethyl, etc.);
(5) aralkyl which may be substituted (e.g., phenyl-C₁₋₄ alkyl such as benzyl, phenethyl, etc.);
(6) formyl or acyl which may be substituted (e.g., C₂₋₄ alkanoyl such as acetyl, propionyl, butyryl, isobutyryl, etc., and C₁₋₄ alkylsulfonyl such as methanesulfonyl, ethanesulfonyl, etc.);
(7) aryl which may be substituted (e.g., phenyl, naphthyl, etc.);
(8) heterocyclic group which may be substituted (e.g., a group formed by eliminating one hydrogen atom from a 5- or 6-membered aromatic heterocyclic ring comprising 1 to 4 heteroatom(s) of 1 or 2 species selected from the nitrogen, sulfur and oxygen atoms (e.g. furan, thiophene, pyrrole, imidazole, pyrazole, thiazole, oxazole, isothiazole, isoxazole, tetrazole, pyridine, pyrazine, pyrimidine, pyridazine, triazine, etc.); and a group formed by eliminating one hydrogen atom from a 5- or 6-membered non-aromatic heterocyclic ring comprising 1 to 4 heteroatom(s) of 1 or 2 species selected from the nitrogen, sulfur and oxygen atoms (e.g. tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, etc.)).

The above-described (1) alkyl which may be substituted, (2) cycloalkyl which may be substituted, (3) alkenyl which may be substituted, (4) cycloalkenyl which may be substituted, (5) aralkyl which may be substituted, (6) acyl which may be substituted, (7) aryl which may be substituted, and (8) heterocyclic group which may be substituted may have one or more substituent(s) (preferably 1 to 3 substituent(s)), including halogen (e.g., fluorine, chlorine, bromine, iodine, etc.); C₁₋₄ alkyl which may be substituted by halogen atom or C₁₋₄ alkoxy; C₁₋₄ alkoxy which may be substituted by halogen atom or C₁₋₄ alkoxy (e.g., methoxy, ethoxy, propoxy, butoxy, trifluoromethoxy, trifluoroethoxy, etc.); C₁₋₄ alkylenedioxy (e.g., -O-CH₂-O-, -O-CH₂-CH₂-O-, etc.); formyl, C₂₋₄ alkanoyl (e.g., acetyl, propionyl, etc.); C₁₋₄ alkylsulfonyl (e.g., methanesulfonyl, ethanesulfonyl, etc.); phenyl-lower (C₁₋₄) alkyl; C₃₋₇ cycloalkyl; cyano; nitro; hydroxy group; thiol group which may be substituted (e.g., thiol, C₁₋₄ alkylthio, etc.); amino group which may be substituted (e.g., amino, mono-C₁₋₄ alkylamino, di-C₁₋₄ alkylamino, 5- or 6-membered cyclic amino such as tetrahydropyrrole, piperazine, piperidine, morpholine, thiomorpholine, pyrrole, imidazole, etc.); carboxyl group; lower (C₁₋₄) alkoxy-carbonyl; lower (C₇₋₁₀) aralkyloxy-carbonyl; carbamoyl; mono-C₁₋₄ alkylcarbamoyl; di-C₁₋₄ alkylcarbamoyl (preferably halogen, lower (C₁₋₄) alkyl which may be halogenated, lower (C₁₋₄) alkoxy which may be halogenated, phenyl-lower (C₁₋₄)alkyl, C₃₋₇ cycloalkyl, cyano or hydroxy group); etc. Particularly, examples of "amino group which may be substituted" represented by R¹ include amino groups which may have one or two alkyl which may be substituted [e.g., C₁₋₁₀ alkyl (such as methyl, ethyl, propyl, isopropyl, butyl, isobutyl, sec-butyl, tert-butyl, pentyl, isopentyl, neopentyl, hexyl, heptyl, octyl, nonyl, decyl, preferably lower (C₁₋₆) alkyl) which may have 1 to 3 substituent(s) selected from the group consisting of halogen (e.g., fluorine, chlorine, bromine, iodine, etc.), nitro, cyano, hydroxy group, thiol group which may be substituted (e.g., thiol, C₁₋₄ alkylthio, etc.), amino group which may be substituted (e.g., amino, mono-C₁₋₄ alkylamino, di-C₁₋₄ alkylamino, 5- or 6- membered cyclic amino such as tetrahydropyrrole, piperazine, piperidine, morpholine, thiomorpholine, pyrrole, imidazole, etc.), carboxyl group which may be esterified or amidated (e.g., carboxyl, C₁₋₄ alkoxycarbonyl, lower (C₇₋₁₀) aralkyloxy-carbonyl, carbamoyl, mono-C₁₋₄ alkylcarbamoyl or di-C₁₋₄ alkylcarbamoyl), C₁₋₄ alkyl which may be substituted by halogen atom or C₁₋₄ alkoxy (e.g., trifluoromethyl, methyl, ethyl, etc.), C₁₋₄ alkoxy which may be substituted by halogen atom or C₁₋₄ alkoxy (e.g., methoxy, ethoxy, trifluoromethoxy, trifluoroethoxy, etc.), C₁₋₄ alkylenedioxy (e.g., -O-CH₂-O-, -O-CH₂-CH₂-O-, etc.), phenyl-lower (C₁₋₄) alkyl, C₃₋₇ cycloalkyl, formyl, C₂₋₄ alkanoyl (e.g., acetyl, propionyl, etc.), C₁₋₄ alkylsulfonyl (e.g., methanesulfonyl, ethanesulfonyl, etc.) or C₁₋₄ alkylsulfinyl (e.g., methanesulfinyl, ethanesulfinyl, etc.), etc.].

Examples of the "cyclic group which may be substituted" represented by R² in the above-described formula include 5- to 8-membered (preferably 5- or 6-membered) saturated or unsaturated alicyclic monocyclic hydrocarbon such as C₅₋₈ cycloalkane (e.g., cyclopentane, cyclohexane, cycloheptane, etc.), C₅₋₈ cycloalkene (e.g., 1-cyclopentene, 2-cyclopentene, 3-cyclopentene, 2-cyclohexene or 3-cyclohexene), C₅₋₈ cycloalkadiene (e.g., 2,4-cyclopentadiene, 2,4-cyclohexadiene, 2,5-cyclohexadiene, etc.); 6-membered aromatic monocyclic hydrocarbon such as benzene; 5- to 8-membered aromatic monocyclic heterocyclic, or saturated or unsaturated non-aromatic monocyclic heterocyclic (aliphatic heterocyclic) ring comprising at least 1 (preferably 1 to 4, and more preferably 1 or 2) of 1 to 3 species (preferably 1 or 2 species) of heteroatoms selected from oxygen, sulfur, and nitrogen atoms; and groups formed by eliminating one hydrogen atom from a condensed ring comprising 2 or 3 identical or different rings selected from the above-described monocyclic rings.

Examples of the "aromatic monocyclic heterocyclic ring" include 5- to 8-membered (preferably 5- or 6-membered) aromatic monocyclic heterocyclic rings (e.g., furan, thiophene, pyrrole, oxazole, isoxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, pyridazine, pyrimidine, pyrazine, triazine, etc.). Examples of "non-aromatic monocyclic heterocyclic ring" include 5- to 8-membered (preferably 5- or 6-membered) saturated or unsaturated monocyclic non-aromatic heterocyclic (aliphatic heterocyclic) rings such as pyrrolidine, tetrahydrofuran, tetrahydrothiophene, thiolane, dithiolane, oxathiolane, pyrroline, imidazolidine, imidazoline, pyrazolidine, pyrazoline, oxazine, oxadiazine, thiazine, thiadiazine, piperidine, morpholine, thiomorpholine, tetrahydropyran, piperazine, pyran, oxepin, thiepin or azepine; and 5- to 8-membered non-aromatic heterocyclic ring comprising any of the above-exemplified aromatic monocyclic heterocyclic rings with all or portion of double bonds therein being saturated.

The "cyclic group which may be substituted" represented by R² may include a group formed by eliminating one hydrogen atom from a condensed ring comprising 2 or 3 (preferably 2) identical or different rings selected from the above-exemplified monocyclic homocyclic rings and heterocyclic rings. Such condensed rings may be saturated, partially saturated or aromatic.

Preferable examples of condensed ring may include those comprising two identical or different heterocyclic rings (preferably one is a heterocyclic ring and the other an aromatic heterocyclic ring, and more preferably two identical or different aromatic heterocyclic rings); and those comprising one heterocyclic ring and one homocyclic ring (preferably one is a heterocyclic ring and the other a benzene ring, and more preferably one is an aromatic heterocyclic ring and the other a benzene ring). Particular examples of such condensed ring are, for example, indole, benzothiophene, benzofuran, benzimidazole, imidazo [1,2-a] pyridine, quinoline, isoquinoline, cinnoline, etc.

Examples of substituent which the "cyclic group which may be substituted" represented by R² may have include those similar to the above-listed substituents which the "hydrocarbon group which may be substituted" that the "benzene ring which may be substituted" represented by A may have may have.

The "cyclic group which may be substituted" represented by R² preferably include 5- or 6-membered cyclic group, 5- or 6-membered aromatic cyclic group, more preferably phenyl, furyl, thienyl, pyrrolyl or pyridyl (preferably 6-membered ring), and most preferably phenyl.

Preferable among compounds having the above-described formula (I) and salts thereof are:
- compounds of the formula (II): 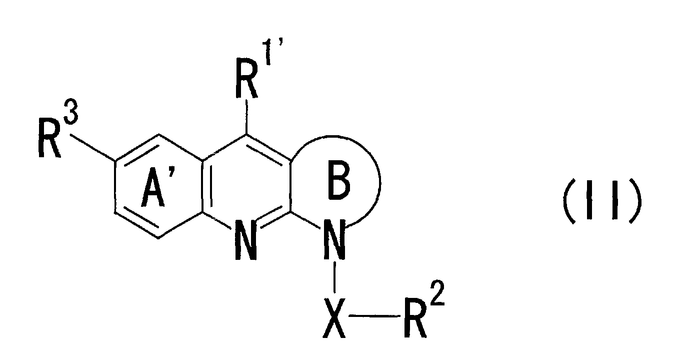 [wherein A' is a benzene ring which may have one or more substituent(s) in addition to substituent R³, B is 5- to 8-membered ring which may be substituted, X is a divalent group containing 1 to 4 atoms in its linear chain, R¹' is an amino group substituted by one or two lower alkyl groups which may be substituted, R² is a cyclic group which may be substituted, and R³ is a hydrocarbon group which may be substituted, a heterocyclic group which may be substituted, a nitro group, a halogen atom, an amino group which may be substituted or a group represented by the formula: R⁴-Y-(wherein Y is an oxygen atom or a sulfur atom which may be oxidized, and R⁴ is a hydrocarbon group which may be substituted or a heterocyclic group which may be substituted)] or salts thereof; and
- compounds of the formula (II'): 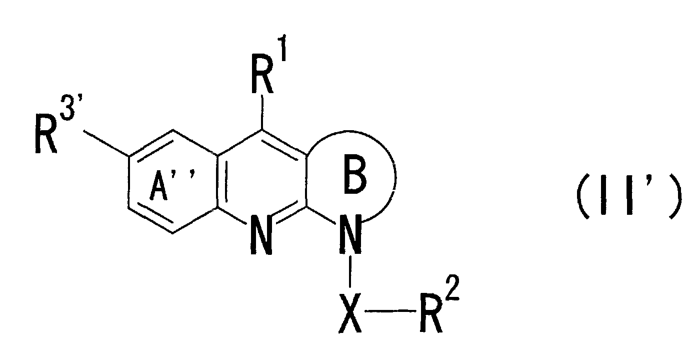 [wherein A'' is a benzene ring which may have one or more substituent(s) in addition to substituent R³', B is 5- to 8-membered ring which may be substituted, X is a divalent group containing 1 to 4 atoms in its linear chain, R¹ is an amino group which may be substituted, R² is a cyclic group which may be substituted, and R³' is a hydrocarbon group which may be substituted, a heterocyclic group which may be substituted, a halogen atom, an amino group which may be substituted or a group represented by the formula: R⁴-Y-(wherein Y is an oxygen atom or a sulfur atom which may be oxidized, and R⁴ is a hydrocarbon group which may be substituted or a heterocyclic group which may be substituted)] or salts thereof.

In the above-described formula, examples of the substituent (excluding substituent R³) which the "benzene ring which may have one or more substituent(s) in addition to substituent R³" represented by A' and the "benzene ring which may which one or more substituent(s) in addition to substituent R³'" represented by A'' may have include, in addition to substituent R³, those similar to the above-exemplified substituents which the "benzene ring which may be substituted" represented by A may have.

Examples of the "substituted amino group" represented by R¹' in the above-described formula include the "amino group which may be substituted" represented by R¹ except for unsubstituted amino group, i.e., amino groups having one or two identical or different substituents similar to the above-exemplified substituents which the "amino group which may be substituted" represented by R¹ may have. Particularly preferable are "amino groups substituted by one or two lower alkyl group(s) which may be substituted".

Examples of "amino group substituted by one or two lower alkyl groups which may be substituted" include amino groups substituted by one or two lower (C₁₋₆) alkyl (e.g., methyl, ethyl, propyl, isopropyl, butyl, isobutyl, sec-butyl, tert-butyl, pentyl, isopentyl, neopentyl, hexyl, etc.) which may have 1 to 3 substituent(s) selected from the group consisting of:
(1) halogen (e.g., fluorine, chlorine, bromine, iodine, etc.);
(2) nitro;
(3) cyano;
(4) hydroxy group;
(5) thiol group which may be substituted (e.g., thiol, C₁₋₄ alkylthio, etc.);
(6) amino group which may be substituted (e.g., amino, mono-C₁₋₄ alkylamino, di-C₁₋₄ alkylamino, 5- or 6-membered cyclic amino such as tetrahydropyrrole, piperazine, piperidine, morpholine, thiomorpholine, pyrrole, imidazole, etc.);
(7) carboxyl group which may be esterified or amidated (e.g., carboxyl, C₁₋₄ alkoxycarbonyl, lower (C₇₋₁₀) aralkyloxy-carbonyl, carbamoyl, mono-C₁₋₄ alkylcarbamoyl, di-C₁₋₄ alkylcarbamoyl, etc.);
(8) C₁₋₄ alkyl which may be substituted by halogen atom or C₁₋₄ alkoxy (e.g., trifluoromethyl, methyl, ethyl, etc.);
(9) C₁₋₄ alkoxy which may be substituted by halogen atom or C₁₋₄ alkoxy (e.g., methoxy, ethoxy, trifluoromethoxy, trifluoroethoxy, etc.);
(10) C₁₋₄ alkylenedioxy (e.g., -O-CH₂-O-, -O-CH₂-CH₂-O-, etc.);
(11) phenyl-lower (C₁₋₄) alkyl;
(12) C₃₋₇ cycloalkyl, formyl or C₂₋₄ alkanoyl (e.g., acetyl, propionyl, etc.);
(13) C₁₋₄ alkylsulfonyl (e.g., methanesulfonyl, ethanesulfonyl, etc.); and
(14) C₁₋₄ alkylsulfinyl (e.g., methanesulfinyl, ethanesulfinyl, etc.).

Those amino groups may be substituted two identical or different substituents.

In the above-described formulae, examples of the "hydrocarbon group which may be substituted" represented by R³ or R³' include those similar to the above-exemplified "hydrocarbon group which may be substituted" which the "benzene ring which may be substituted" represented by A may have.

In the above-described formulae, examples of " heterocyclic group which may be substituted" represented by R³ or R³ include those similar to the above-exemplified "heterocyclic group which may be substituted" which the "benzene ring which may be substituted" represented by A may have.

In the above-described formulae, examples of "amino group which may be substituted" represented by R³ or R³' include those similar to the above-exemplified "amino group which may be substituted" which the "benzene ring which may be substituted'' represented by A may have.

In the above-described formulae, examples of the "hydrocarbon group which may be substituted" or "heterocyclic group which may be substituted" represented by R⁴ in the group of the formula: R⁴-Y- include those similar to the above-exemplified "hydrocarbon group which may be substituted" or "heterocyclic group which may be substituted" which the "benzene ring which may be substituted" represented by A may have.

In the above-described formulae, examples of the " sulfur atom which may be oxidized" represented by Y in the group of the formula R⁴-Y- include S, S(O) and S(O)₂.

Examples of salts of compounds having GPR 14-antagonizing activity to be used in the present invention [including compounds represented by formula (I), (II) or (II')] preferably include pharmaceutically acceptable salts such as salts with inorganic base, organic base, inorganic acid, organic acid, or basic or acidic amino acid.

Preferable examples of salts with inorganic base include alkaline metal salts such as sodium salts or potassium salts; alkaline earth metal salts such as calcium salts or magnesium salts; and aluminium salts and ammonium salts, etc.

Preferable examples of salts with organic base include salts with, for example, trimethylamine, triethylamine, pyridine, picoline, ethanolamine, diethanolamine, triethanolamine, dicyclohexylamine or N,N'-dibenzylethylenediamine, etc.

Preferable examples of salts with inorganic acid include salts with, for example, hydrochloric acid, hydrobromic acid, nitric acid, sulfuric acid or phosphoric acid, etc.

Preferable examples of salts with organic acid include salts with, for example, formic acid, acetic acid, trifluoroacetic acid, fumaric acid, oxalic acid, tartaric acid, maleic acid, citric acid, succinic acid, malic acid, methansulfonic acid, benzenesulfonic acid or p-toluenesulfonic acid, etc.

Preferable examples of salts with basic amino acid include salts with, for example, arginine, lysine or ornithine, etc. Preferable examples of salts with acidic amino acid include salts with, for example, aspartic acid or glutamic acid, etc.

Compounds having GPR 14-antagonizing activity to be used in the present invention [including compounds represented by formula (I), (II) and (II')] may be hydrates or non-hydrates. Compounds having GPR 14-antagonizing activity to be used in the present invention [including compounds represented by formula (I), (II) and (II')] can be individually isolated by any known means for separation/purification as desired when they are present as configurational isomers, diastereoisomers or conformers. Compounds having GPR 14-antagonizing activity to be used in the present invention [including compounds represented by formula (I), (II) and (II')] can be separated into S-form and R-form by any conventional optical resolution means when they are present as racemic modifications. All of those optically active substances and racemic modifications are encompassed by the present invention.

Compounds having GPR 14-antagonizing activity to be used in the present invention and salts thereof [including compounds represented by formula (I), (II) and (II') and salts thereof] [hereinafter sometimes referred to as GPR14 antagonist] may be use as prodrugs. Examples of such prodrug may include compounds which may be converted into GPR14 antagonist through, for example, enzyme- or gastric acid-mediated reaction *in vivo* under physiological conditions, i.e., compounds which may be enzymatically oxidized, reduced and/or hydrolyzed to be converted into GPR14 antagonist, and compounds which may be hydrolyzed by gastric acid and the like to be converted into GPR14 antagonist. Examples of prodrug of GPR14 antagonist include compounds comprising GPR 14 antagonist in which amino group or groups have been acylated, alkylated or phosphorylated (e.g., compounds comprising GPR14 antagonist in which amino group or groups have been eicosanoylated, alanylated, pentylamino carbonylated, (5-methyl-2-oxo-1,3-dioxolene-4-yl) methoxycarbonylated, tetrahydrofuranylated, pyrrolidylmethylated, pivaloyloxymethylated, or tert-butylated); compounds comprising GPR 14 antagonist in which hydroxy group or groups have been acylated, alkylated, phosphorylated or borated (e.g., compounds comprising GPR14 antagonist in which hydroxy group or groups have been acetylated, palmitoylated, propanoylated, pivaloylated, succinylated, fumarylated, alanylated or dimethylamino methylcarbonylated); compounds comprising GPR 14 antagonist in which carboxyl group or groups have been esterified or amidated (e.g., compounds comprising GPR 14 antagonist in which carboxyl group or groups have been ethylesterified, phenylesterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolene-4-yl)methyl esterified, cyclohexyloxycarbonylethyl esterified or methylamidated, etc.). These compounds can be prepared from GPR14 antagonist using any known method.

Further, prodrugs of GPR14 antagonist may be compounds which may be converted into GPR14 antagonist under physiological conditions as described in "Development of pharmaceuticals (Iyakuhinn no Kaihatsu)", vol. 7, Molecular Design pp.163-198, Hirokawa Shoten (1990).

GPR14 antagonist may be labeled with any suitable isotope such as ³H, ¹⁴C, ³⁵S, ¹²⁵I, etc.

GPR14 antagonist according to the present invention may be used alone or in combination with pharmaceutically acceptable carrier or carriers, to formulate solid (such as tablet, capsule, granule or powder) or liquid (such as syrup or injection) formulations which can then be administered orally or parenterally.

Dosage forms for parenteral administration include, for example, injection, instillation and suppository.

Examples of pharmaceutically acceptable carrier include various organic or inorganic carrier materials which have been conventionally used as formulation bases. Excipient, lubricant, binder and/or disintegrator may be used for solid formulations while solvent, dissolution adjuvant, suspending agent, isotonizing agent, buffer and/or soothing agent may be used for liquid formulations. Additive or additives may be added when required, including preservative, anti-oxidant, colorant and/or sweetening agent. Preferable examples of excipient include lactose, saccharose, D-mannitol, starch, crystalline cellulose or light anhydrous silicic acid, etc. Preferable examples of lubricant include, for example, magnesium stearate, calcium stearate, talc or colloidal silica, etc. Preferable examples of binder include, for example, crystalline cellulose, saccharose, D-mannitol, dextrin, hydroxypropylcellulose, hydroxypropylmethylcellulose, polyvinylpyrrolidone, etc. Preferable examples of disintegrator include, for example, starch, carboxymethyl cellulose, carboxy methylcellulose calcium, crosscarmellose sodium or sodium carboxymethyl starch. Preferable examples of solvent include, for example, water for injection, alcohol, propylene glycol, macrogol, sesame oil or corn oil. Preferable examples of dissolution adjuvant include, for example, polyethylene glycol, propylene glycol, D-mannitol, benzyl benzoate, ethanol, trisaminomethane, cholesterol, triethanolamine, sodium carbonate or sodium citrate. Preferable examples of suspending agent include: surfactants such as stearyl triethanolamine, sodium lauryl sulfate, laurylamino propionate, lecitin, benzalkonium chloride, benzethonium chloride or glyceryl monostearate; and hydrophilic polymers such as polyvinyl alcohol, polyvinylpyrrolidone, sodium carboxymethylcellulose, methylcellulose, hydroxymethyl cellulose, hydroxyethyl cellulose, hydroxypropyl cellulose, etc. Preferable examples of isotonizing agent include, for example, sodium chloride, glycerine, D-mannitol, etc. Preferable examples of buffer include buffer solution of, for example, phosphate, acetate, carbonate, citrate, etc. Preferable examples of soothing agent include, for example, benzyl alcohol, etc. Preferable examples of preservative include, for example, p-hydroxybenzoic esters, chlorobutanol, benzyl alcohol, phenethyl alcohol, dehydroacetic acid and sorbic acid. Preferable examples of anti-oxidant include, for example, sulfite and ascorbic acid, etc.

The preparation of compounds represented by formula (I) [including compounds represented by formula (II) and (II') having a novel structure] or salts thereof will be described below.

Compounds represented by formula (I) or salts thereof can be prepared according to any conventionally known method. Particularly, compounds represented by formula (I) or salts thereof can be prepared according to or substantially according to the methods described below or in "Tetrahedron Letters", vol. 40, pp.5643-5646, Japanese Patent Application Laid-Open No. 3-220189 or Japanese Patent Publication No. 48-30280.

Compounds which may be used in the following methods may form salts similar to compounds (I) provided that they do not have any adverse effect on the reactions.

In the reactions described below, when the starting materials have amino, carboxyl, and/or hydroxy group(s) as substituent or substituents, protecting group or groups which are typically used in peptide chemical may be introduced into the substituent or substituents. In this case, the protecting group or groups may be removed as required after reaction to obtain title compounds.

Examples of protecting group for amino group may include, C₁₋₆ alkylcarbonyl which may have one or more substituent(s) (e.g., acetyl, propionyl, etc.), formyl, phenylcarbonyl, C₁₋₆ alkyloxycarbonyl (e.g., methoxycarbonyl, ethoxycarbonyl, t-butoxycarbonyl, etc.), phenyloxycarbonyl (e.g., benzoxycarbonyl, etc.), C₇₋₁₀ aralkyloxycarbonyl (e.g., benzyloxycarbonyl, etc.), trityl, phthaloyl, etc. Those may have about 1 to 3 substituent(s), including halogen atom (e.g., fluorine, chlorine, bromine, iodine, etc.), C₁₋₆ alkylcarbonyl (e.g., acetyl, propionyl, butyryl, etc.) and nitro group.

Examples of protecting group for carboxyl group may include C₁₋₆ alkyl which may have one or more substituent(s) (e.g., methyl, ethyl, propyl, isopropyl, butyl, tert-butyl, etc.), phenyl, trityl, silyl, etc. They may be have about 1 to 3 substituent(s), including halogen atom (e.g., fluorine, chlorine, bromine, iodine, etc.), C₁₋₆ alkylcarbonyl (e.g., acetyl, propionyl, butyryl, etc.), formyl and nitro group, etc.

Examples of protecting group for hydroxy group may include C₁₋₆ alkyl which may have one or more substituent(s) (e.g., methyl, ethyl, propyl, isopropyl, butyl, tert-butyl, etc.), phenyl, C₇₋₁₀ aralkyl (e.g., benzyl, etc.), C₁₋₆ alkylcarbonyl (e.g., acetyl, propionyl, etc.), formyl, phenyloxycarbonyl, C₇₋₁₀ aralkyloxycarbonyl (e.g., benzyloxycarbonyl, etc.), pyranyl, furanyl, silyl, etc. They may have about 1 to 4 substituents, including halogen atom (e.g., fluorine, chlorine, bromine, iodine, etc.), C₁₋₆ alkyl, phenyl, C₇₋₁₀ aralkyl and nitro group, etc.

Protecting groups may be introduced and/or removed according to or substantially according to any conventionally known methods [e.g., methods described in J.F.W.McOmie et al., "Protective groups in organic chemistry" Prenum Press]. Preferably, protecting groups may be removed by, for example, treating with acid, base, reduction, UV-light, hydrazine, phenylhydrazine, sodium N-methyldithiocarbamate, tetrabutyl ammonium fluoride or palladium acetate, etc.

### Preparation

Among compounds represented by formula (I) [including compounds represented by formula (II) or (II') having a novel structure] and salts thereof, compounds represented by formula (Ia) in which R¹ is unsubstituted amino group or salts thereof can be prepared according to, for example, the following scheme: 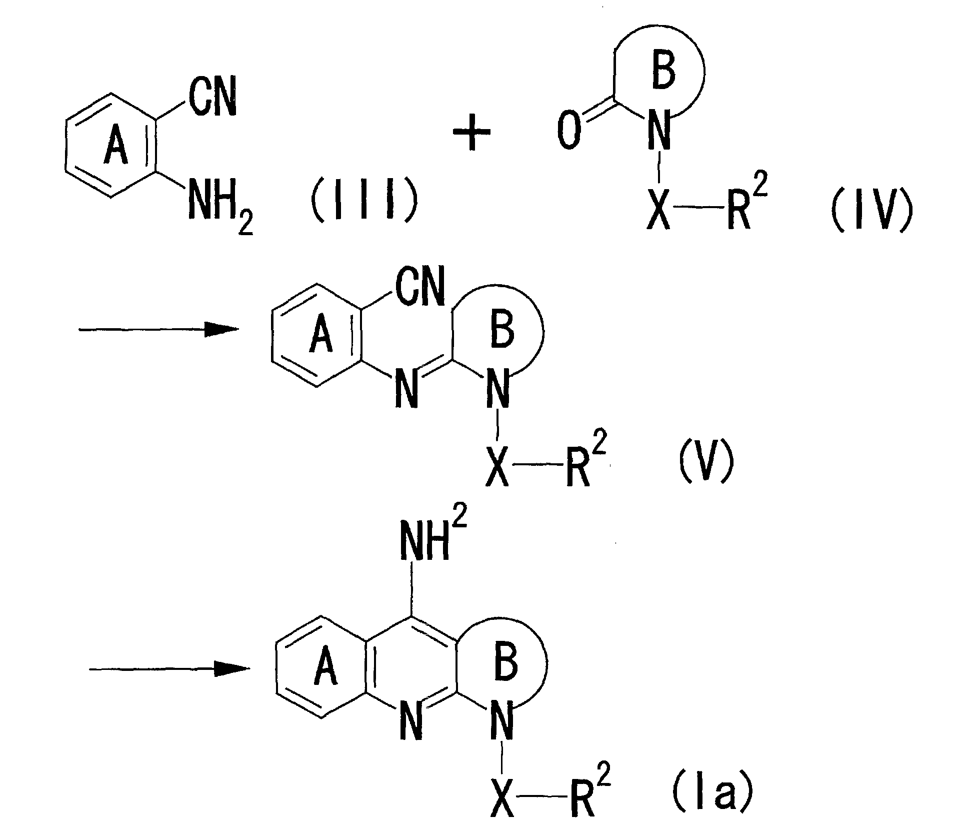 [for definitions, see above].

Compounds represented by formula (Ia) or salts thereof can be obtained by allowing compound represented by formula (III) or salts thereof to react with compound represented by formula (IV) or salts thereof to obtain product compound represented by (V) or salts thereof, and then cyclizing the product compound according to or substantially according to the methods described in Japanese Patent Application Laid-Open No. 3-220189, Japanese Patent Publication No. 48-30280 or other publications.

Alternatively, compounds represented by formula (I) [including compounds represented by formula (II) or (II') having a novel structure] or salts thereof can also be prepared according to, for example, the following scheme: 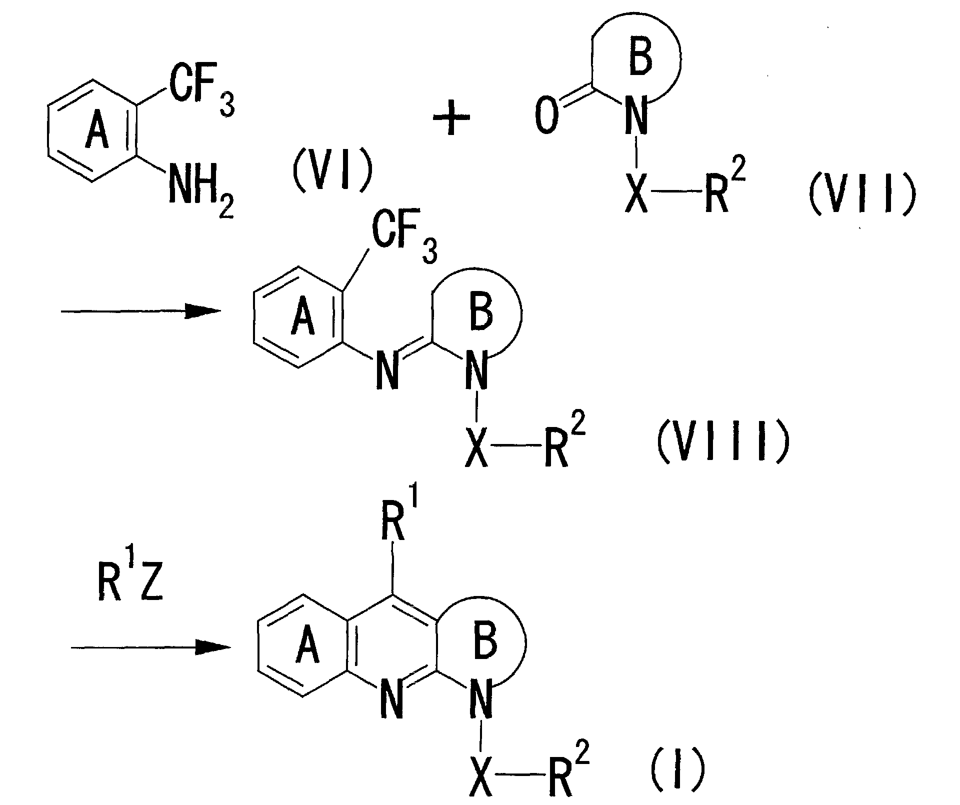 [wherein Z represents alkaline metal, and for other definitions, see above].

Compound represented by formula (VI) or salts thereof may be allowed to react with compound represented by formula (VII) or salts thereof to give product compound represented by formula (VIII) or salts thereof which may be then allowed to react with compound represented by formula R¹Z, to prepare compound represented by formula (I) or salts thereof, according to or substantially according to the method described in, for example, "Tetrahedron letters", vol. 40, pp.5643-5646.

Examples of alkaline metal represented by Z include lithium, sodium, etc.

Reaction may be performed without solvent or in solvent. Any suitable solvent may be used provided that the solvent does not have any adverse effect on the reaction, including, for example, ether solvent (e.g., diethyl ether, tetrahydrofuran, dioxane, etc.), halogen solvent (e.g., dichloromethane, dichloroethane, chloroform, carbon tetrachloride, etc.), hydrocarbon solvent (e.g., benzene, toluene, hexane, heptane, etc.), amide solvent (dimethylformamide, dimethylacetamide, N-methylpyrrolidone, etc.), ester solvent (ethyl acetate, methyl acetate, etc.), acetonitrile, dimethylsulfoxide, etc. Solvent may be used alone or in combination.

About 0.5-20 molar equivalent, and preferably about 0.8-10 molar equivalent of compound represented by the formula R¹Z may be allowed to react with compound represented by formula (VIII) or salts thereof at about - 80°C to 200°C (preferably about -80°C to 80°C) for about 0.1 to 96 hours (preferably about 0.5 to 72 hours).

Compounds represented by formula (I) or salts thereof in which R¹ is not an unsubstituted amino group can be prepared substantially according to any known method. For example, such compounds or salts thereof can be prepared by converting compounds represented by formula (Ia) or salts thereof (starting materials) obtained in the above-described scheme according to the following reactions: 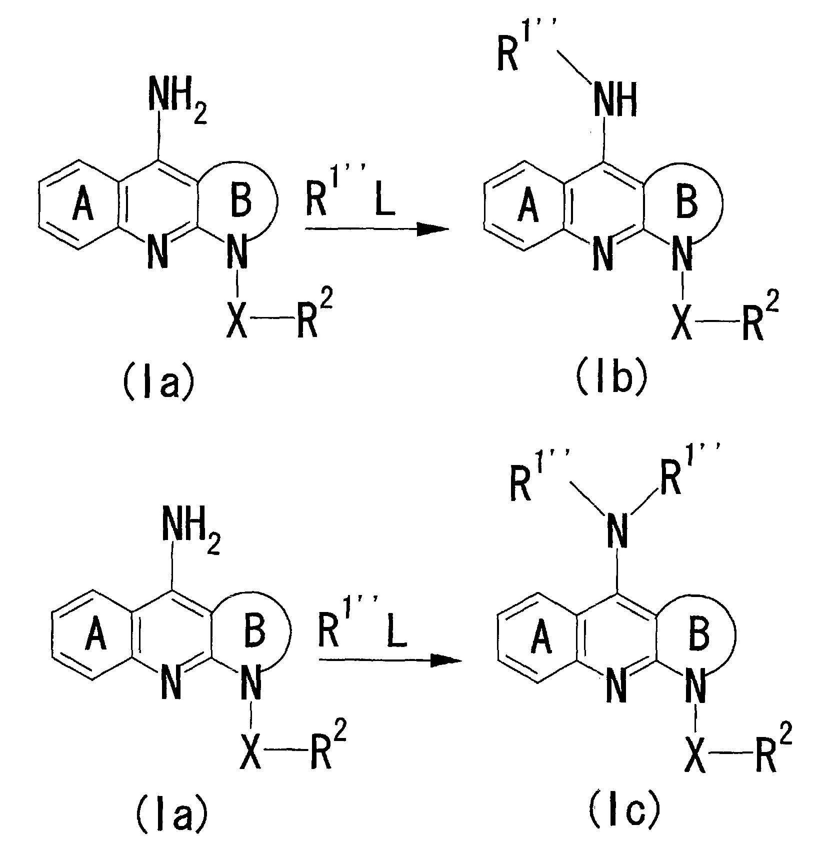 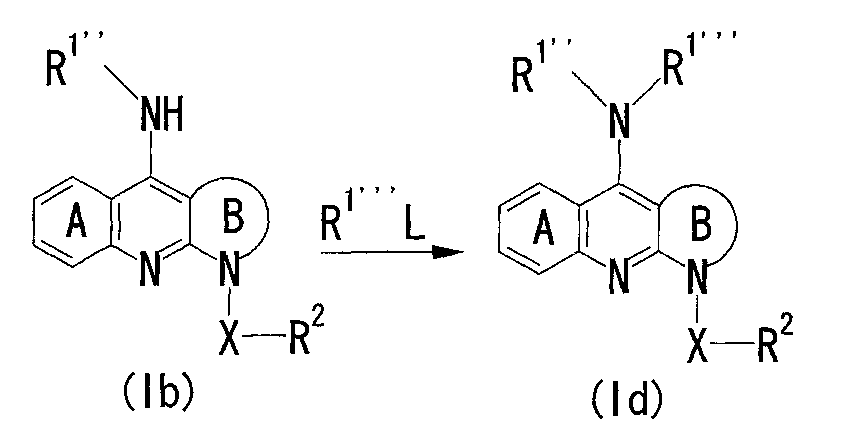 [wherein R¹'' and R¹''' independently represent amino group substituents (preferably lower alkyl group which may be substituted) and L is a leaving group].

Examples of leaving group represented by L include halogen atoms such as chlorine, bromine, iodo atom, etc., and sulfonate ester such as methanesulfonyl group, toluenesulfonyl group, etc.

Reaction may be performed without solvent or in solvent. Any suitable solvent may be used provided that the solvent does not have any adverse effect on the reaction, including, for example, ether solvent (e.g., diethyl ether, tetrahydrofuran, dioxane, etc.), halogen solvent (e.g., dichloromethane, dichloroethane, chloroform, carbon tetrachloride, etc.), hydrocarbon solvent (e.g., benzene, toluene, hexane, heptane, etc.), amide solvent (dimethylformamide, dimethylacetamide, N-methylpyrrolidone, etc.), ester solvent (ethyl acetate, methyl acetate, etc.), acetonitrile, dimethylsulfoxide, etc. Solvent may be used alone or in combination. Optionally, reaction may be performed in the presence of base (e.g., triethylamine, 4-(dimethylamino)pyridine, 2-tert-butylimino-2-diethylamino-1,3-dimethylperhydro-1,3,2-diazaphospholine, sodium hydrogencarbonate, potassium hydrogencarbonate, sodium carbonate, potassium carbonate, sodium hydride or potassium hydride), or phase transfer catalyst (e.g., quaternary ammonium salt such as tetrabutylammonium bromide, benzyl triethyl ammonium chloride, etc., and crown ethers such as 18-crown-6, etc.), or both base and phase transfer catalyst.

About 0.5-20 molar equivalent, and preferably about 0.8-10 molar equivalent of compound of the formula: R¹''L or R¹'''L may be allowed to react with compound represented by formula (Ia) or salt thereof or by formula (Ib) or salt thereof, respectively, at about -20°C to 200°C (preferably about 20°C to 150°C) for about 0.1 to 96 hours (preferably about 0.5 to 72 hours). Base may typically be used at an amount of about 0.5 to 10 molar equivalent, and preferably about 1 to 5 molar equivalent, relative to compound represented by formula (Ia) or (Ib).

For compounds represented by any of formulae (Ia)-(Id) or salts thereof in which ring A is substituted by a halogen atom such as chlorine, bromine, iodine, etc., the substituent may be easily replaced by any other functional group (e.g., substituents which the benzene ring represented by ring A may have) according to any known substitution reaction (e.g., Suzuki coupling reaction, Still reaction, Heck reaction, etc.).

Compounds (I) obtained as described above can be isolated and purified by any known means for separation/purification such as concentration, concentration under reduced pressure, solvent extraction, crystallization, recrystallization, dissolution, or chromatography, etc.

Since compounds having GPR 14-antagonizing activity or salts thereof according to the present invention [including compounds represented by formula (I), (II) and (II') or salts thereof] have a potent GPR 14-antagonizing activity, those can be used as therapeutic agents for expressing various vasoactivities (such as accentuation) or inhibition of vasoconstriction), and preferably as vasoconstriction inhibitors.

Compounds having GPR 14-antagonizing activity or salts thereof according to the present invention [including compounds represented by formula (I), (II) and (II') or salts thereof] can be used as therapeutic agents for preventing/treating various diseases (e.g., circulatory system-associated diseases), more preferably hypertension, arteriosclerosis, hypertension, hypercardia, myocardial infarction, heart failure or septic shock, and most preferably ischemic myocardial infarction or congestive heart failure.

Further, compounds having GPR 14-antagonizing activity or salts thereof according to the present invention [including compounds represented by formula (I), (II) and (II') or salts thereof] have very low toxicity and thus can be used safely.

Dose of compounds having GPR 14-antagonizing activity or salts thereof according to the present invention may depend on various factors such as the condition and weight of the patient to be treated and administration manner. For oral administration, compound may be preferably administered at an amount of about 0.1-100mg [active agent (e.g., compound represented by formula (II) or (II') or salt thereof)]/adult (50kg)/day, more preferably about 1-50mg (active agent)/adult (50kg)/day, and most preferably about 1-20mg (active agent)/adult (50kg)/day, once to three times a day.

Compounds having GPR 14-antagonizing activity or salts thereof according to the present invention [including compounds represented by formula (I), (II) and (II') or salts thereof] may be used in combination with other therapeutic agent or agents (particularly with therapeutic agent for preventing/treating hypertension). In this case, these agents may separately be formulated into different preparations, or may be formulated together into one preparation, by blending with any pharmaceutically acceptable carrier, excipient, binder and/or diluent, and then administered orally or parenterally. When these agents are separately formulated into different preparations, these preparations may be administered to a subject after mixing together by using diluent just prior to use. Alternatively, these preparations may separately be administered to the subject simultaneously or with a certain time interval. A kit product for mixing separate preparations using diluent and the like just prior to use for administration (e.g., a kit for injection which contains two or more ampoules each containing a different powdery drug and a diluent for mixing the drugs just prior to use) as well as a kit product for administering separate preparations to a subject simultaneously or separately with a certain time interval (e.g., a kit for administering two or more types of separate tables to a subject simultaneously or separately with a certain time interval wherein tablets each containing a different drug are packed in the same bag or different bags, and a column is provided on the bag in which a time interval for drug administration can be written) are encompassed by the pharmaceutical compositions of the present invention.

Particular examples of other therapeutic agents which can be used in combination with compounds having GPR 14-antagonizing activity or salts thereof according to the present invention include:
- drugs for treating hypertension such as diuretic [e.g., furosemide (Lasix), bumetanide (Lunetoron) or azosemide (Diart) ], antihypertensive drug [e.g., ACE inhibitor such as enalapril maleate (Renivace) or delapril hydrochloride] and Ca antagonist (manidipine or amlodipine), or α- or β-receptor blocker];
- drugs for treating chronic heart failure such as cardiotonic drug [e.g., cardiotonic glycoside (e.g., digoxin), β-receptor stimulator (catecholamine preparation such as denopamine or dobutamine) and PDE inhibitor], diuretic [e.g., furosemide (Lasix) or spironolactone (Aldactone) ], ACE inhibitor [e.g., enalapril maleate (Renivace)], Ca antagonist [e.g., amlodipine] and β-receptor blocker;
- antiarrhythmic drugs such as disopyramide, lidocaine, quinidine sulfate, flecainide acetate, mexiletine hydrochloride, amiodarone hydrochloride, as well as β-blocker, Ca antagonist;
- drugs for preventing/treating thrombogenesis: coagulation inhibitor [e.g., heparin sodium, heparin calcium, warfarin calcium (warfarin), blood coagulation factor Xa inhibitor and drugs capable of balancing coagulation fibrinolytic system], thrombolytic agent [e.g., tPA, urokinase, prourokinase, etc.], antiplatelet drug [e.g., aspirin, sulfinpyrazolo (Anturan), dipyridamole (Persantin), ticlopidine (Panaldine), cilostazol (Pletaal) and GP IIb/IIIa antagonist (ReoPro)];
- coronary vasodilators such as nifedipine, diltiazem, nicorandil or nitrite agent; and
- cardioplegic drugs such as opener for cardiac ATP-K, Na-H exchange inhibitor, endothelin antagonist or urotensin antagonist.

### Best Mode for Carrying out the Invention

Although the present invention will be described in more detail by referring to Experimental Examples, Preparation Examples, Reference Example and Synthesis Examples, these examples are provided to illustrate the invention but not to limit its scope.

Brief description of SEQ ID NOS used herein will be provided below:

### [SEQ ID NO: 1]

A synthetic DNA used for screening cDNA encoding human GPR14 protein.

### [SEQ ID NO: 2]

Another synthetic DNA used for screening cDNA encoding human GPR14 protein.

### [SEQ ID NO: 3]

The entire nucleotide sequence of cDNA encoding human GPR14 protein with nucleotide sequences which may be recognized by restriction enzymes Sal I and Spe I added at the 5'- and 3'-termini, respectively.

### [SEQ ID NO: 4]

The amino acid sequence of human GPR14 protein confirmed in Reference Example 2.

### Examples

### Reference Example 1: Amplifying cDNA for human GPR14 receptor by PCR method using human skeletal muscle-derived cDNA

PCR amplification was performed by using cDNA derived from human skeletal muscle (Clontech) as a template and two synthetic DNA primers (SEQ ID NOS: 1 and 2). The synthetic DNA primers were designed so that the gene in the region which is to be translated into receptor protein would be amplified, and such that nucleotide sequences which may be recognized by restriction enzymes Sal I and Spe I were added at the 5'- and 3'-termini of the gene, respectively. Reaction solution included 2.5µl of cDNA template, synthetic DNA primers (0.2µM each), 0.2mM dNTPs, 1µl of Advantage 2 polymerase mix (Clontech) and the buffer appended to the enzyme (total reaction volume of 50µl). Thermocycler (Perkin-Elmer Corp.) was used for amplification. The amplification cycle consisted of heating at 95°C for 60 seconds, followed by 5 rounds of 95°C for 30 seconds and 72°C for 3 minutes, 5 rounds of 95°C for 30 seconds and 70°C for 3 minutes, and then 20 rounds of 95°C for 30 seconds and 68°C for 3 minutes, and finally heating at 68°C for 3 minutes. The resultant PCR amplification products were confirmed by purification by electrophoresis on a 0.8% agarose gel followed by staining with ethidium bromide.

### Reference Example 2: Subcloning of PCR product into plasmid vector and confirming amplified cDNA by reading the nucleotide sequence of cDNA insert

PCR reaction products obtained in Reference Example 1 were separated on a 0.8% low-melting agarose gel, a gel containing bands was excised using a razor, and DNA was collected using GENECLEAN SPIN (BIO 101, Inc.). According to the prescription included in Eukaryotic TOPO™ TA Cloning kit (Invitrogen), the collected DNA was cloned into a plasmid vector for expression in animal cells, pcDNA3.1/V5/His, to construct a plasmid for protein expression, pcDNA3.1-hGPR14 which was then introduced into *Escherichia coli* DH5 α competent cells (Toyobo Co., Ltd.) for transformation. Then, clone which contained cDNA insert fragment was selected on an ampicillin-containing LB agar medium, and separated using a sterilized toothpick to obtain transformant *E. coli* DH5 α/pcDNA3.1-hGPR14. Each clone was cultured overnight on an ampicillin-containing LB medium, and Quiawell 8 Ultra Plasmid kit (Qiagen) was used to prepare plasmid DNA. Portion of DNA prepared was digested with restriction enzyme Sal I, and the size and direction of receptor cDNA fragment inserted were determined. The sequences of nucleotides were determined by using DyeDeoxy Terminator Cycle Sequence Kit (Perkin-Elmer Corp.) and then reading in a fluorescence automatic sequencer. The sequence of clone obtained was analyzed and confirmed to be consistent with a genetic sequence comprising the sequence of human GPR14 gene, of which entire sequence has been reported (EP 0 859 052 A1), and Sal I and Spe I recognition sequences added to the 5'- and 3'-termini of the sequence, respectively (SEQ ID NOS: 3 and 4). It should be noted that although the 1133rd base in the sequence of human GPR14 gene (SEQ ID NO: 3) was identified as C in the report (EP 0 859 052 A1) while it was identified as G in the present Example though the amino acids which would be translated from these sequences may be the same.

### Reference Example 3: Preparing human GPR14-expressing CHO cell

After the transformant *E. coli* DH5α/pcDNA3.1-hGPR14 prepared in Reference Example 2 was cultured, plasmid DNA for pcDNA3.1-hGPR1 was prepared by using Plasmid Midi Kit (Qiagen). The plasmid DNA was introduced into CHO dhfr cells using CellPhect Transfection Kit (Amersham Pharmacia Biotech) according to the protocol appended thereto. 10µg of DNA was co-precipitated with calcium phosphate to prepare a suspension which was then added to a 10cm petri dish on which 5 x 10⁵ or 1 x 10⁶ CHO dhfr⁻ cells had previously been inoculated 24 hours before then. Cells were cultured in a MEMα medium containing 10% fetal bovine serum for one day, subcultured, and cultured in a selection medium, a MEMα medium containing 0.4 mg/ml G418 (GIBCO BRL) and 10% dialysis fetal bovine serum. Colonies of transformed cells (CHO/hGPR14), which were human GPR14-expessing CHO cells growing in the selection medium, were selected.

### Experimental Example 1: Preparing human GPR14-expressing cell fraction

To 1 x 10⁸ CHO/GPR14 cells were added 10 ml of homogenate buffer (10 mM NaHCO₃, 5 mM EDTA, 0.5 mM PMSF, 1µg/ml pepstatin, 4µg/ml E64, 20µg/ml leupeptin), and disrupted using Polytron (12,000 rpm, 1 minute). Cell debris solution was centrifuged at 1,000g for 15 minutes to obtain a supernatant. The supernatant was then ultra-sonicated (in Beckman type 30 rotor, 30,000 rpm, 1 hour), and the resultant precipitant was collected as human GPR14-expressing CHO cell fraction.

### Experimental Example 2: Preparing isotope-labeled human urotensin II

Isotope-labeled human urotensin II to be used in experiments for testing inhibition of binding was prepared as described below. 5 µg of human urotensin II (available from Peptide Institute, Inc.) was dissolved in 25µl of 0.4M sodium acetate (pH 5.6). To the solution was added 200ng of lactoperoxidase (Wako Pure Chemical Industries, Ltd.) followed by 1 mCi [¹²⁵I]-sodium iodide (Amersham Pharmacia Biotech) and 200 ng of hydrogen peroxide (10µl). The solution was left to stand at room temperature for 10 minutes, another 200 ng of hydrogen peroxide (10µl) was added thereto and then the solution was left to stand for 10 minutes. The mixture was then purified by HPLC using TSKgel ODS-80Tₛ column (4.6 mm x 25 cm, Toso Co., Ltd.) to obtain [¹²⁵I]-labeled human urotensin II.

### Experimental Example 3: Experiment for testing the ability of test compound to inhibit binding of urotensin II to GPR14 using human GPR14-expressing cell fraction and isotope-labeled urotensin II

Human GPR14-expressing CHO cell fraction was diluted in a membrane diluting buffer (20mM phosphate buffer (pH7.3), 150mM NaCl, 5mM MgCl₂,0.1% BSA, 0.05% CHAPS, 0.5mM PMSF, 0.1µg/ml Pepstatin, 20µg/ml Leupeptin, 4µg/ml E-64) to prepare a solution of cell membrane fraction (protein concentration:3µg/ml) for assay. The membrane fraction solution for assay was dispensed in 96-well microplates (85µl each) which were left for stand for reaction at 25°C for 3 hours after adding: 10µl of membrane diluting buffer containing 1nM [¹²⁵I]-labeled human urotensin II and 5µl of di-methylsulfoxide diluted 5-times (by volume) in membrane diluting buffer for examining the total binding; 10µl of membrane diluting buffer containing 1nM [¹²⁵I]-labeled human urotensin II and 5µl of 20% dimethylsulfoxide-containing membrane diluting buffer containing 20µM□human urotensin II without isotope-labeling for examining non-specific binding; and 5µl of a solution of test compound in dimethylsulfoxide diluted 5-times (by volume) in membrane diluting buffer and 10µl of membrane diluting solution containing 1nM [¹²⁵I]-labeled-human urotensin II for testing the ability of test compounds to inhibit binding. The mixture solution was filtrated through a filter plate (GF/C, Watman). Next, the filter was washed three times with membrane diluting buffer (0.2ml), added with 20µl of Microscinti 20 (Packard), and determined for radioactivity in Topcount (Packard). Specific-binding is calculated by subtracting non-specific binding from the total binding. The ability of test compound to inhibit binding of urotensin II to human GPR14 is represented by the ratio of [(total binding) - (the radio activity of the cell fraction to which test compound was added)] vs [specific binding]. Concentrations of test compounds at which the compounds showed 50% inhibition of human GPR14 binding activity are shown. Test compounds 1 and 3 were purchased from ASINEX, and test compound 2 was purchased from CHEMBRIDGE.

Results are shown in Table 1 below.

**[Table 1]**

| Test compound | Inhibitory concentration |
|---|---|
| 1 | 4.3nM |
| 2 | 2.4nM |
| 3 | 33nM |

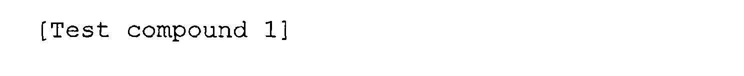

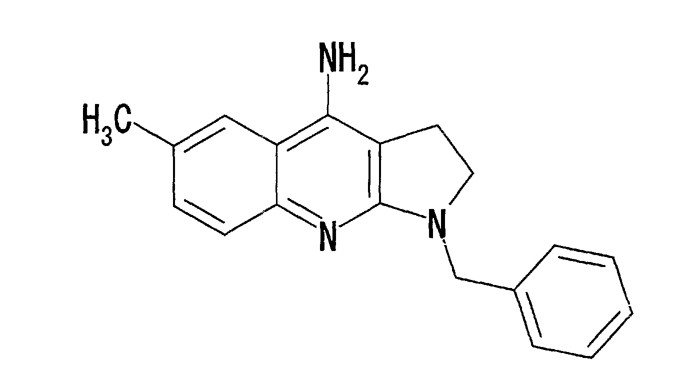

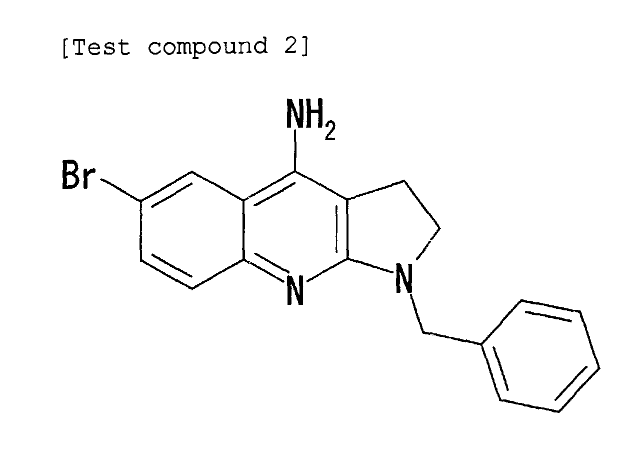

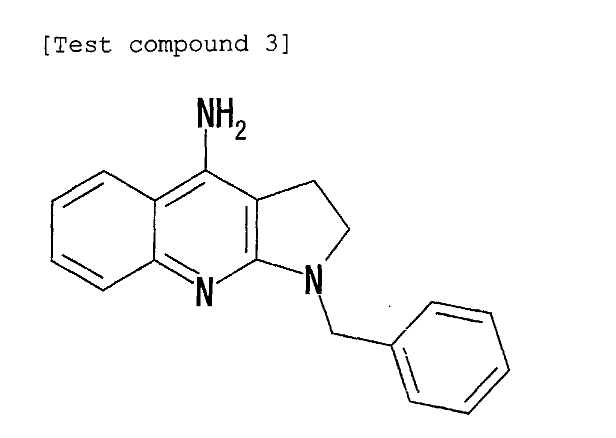

### Experimental Example 4: Change in calcium concentration in human GPR14-expressing CHO cell caused by test compound

GPR14-expressing CHO cells were inoculated on a 96-well plate at 1×10⁴ cell/well, cultured for 48 hours, and then washed with 0.1ml of HBSS containing 20mM HEPES(pH7.4), 1% FCS and 1% penicillin-streptomycin (hereinafter referred to as "wash buffer"). Next, 100µl of another wash buffer containing 4µM Fluo3, 0.04% pluronic acid and 2.5mM probenicid (hereinafter referred to as "reaction buffer") was added thereto for reaction at 37°C for 1 hour. The reaction buffer was then removed and the plate was washed three times with 0.2ml of wash buffer. Then, 90µl of wash buffer and 10µl of a solution of test compound in dimethylsulfoxide diluted 10 times (by volume) in membrane diluting buffer were added for agonist activity assay, while, for antagonist activity assay, furthermore 10µl of 10nM urotensin II was additionally added to determine change in intracellular calcium concentration in FLIPR (Japan Molecular Device). Both of test compounds 1 and 2 inhibited urotensin II-induced increase in intracellular calcium concentration.

### Synthesis Example

### Synthesis Example 1: 1-benzyl-6-bromo-2,3-dihydro-1H-pyrrolo [2,3-b] quinoline-4-ylamine

N-benzylpyrrolidone (1.8g, 10.4mmol) was dissolved in 4ml of chloroform, and phosphorus oxychloride (1.8g, 11.7mmol) was added to the solution which was then stirred at room temperature for 30 minutes. To the mixture 4-bromo-2-cyanoaniline (2.0g, 10mmol) was added and the mixture was refluxed for 3 hours while heating. The reaction solution was poured into an ice-water and neutalized with 20% aqueous sodium hydroxide solution. After extraction with chloroform, the organic layer was dried over anhydrous sodium sulfate. After concentration under reduced pressure, the residue was dissolved in 10 ml of nitrobenzene. 2g of zinc chloride was added thereto and then the mixture was heated at 160°C for 3 hours. 20% aqueous sodium hydroxide solution was added to the reaction mixture and the reaction mixture was extracted with ethyl acetate. The organic layer was dried over anhydrous sodium sulfate and then concentrated under reduced pressure. The resultant residue was subjected to column chromatography on silica gel (50g of silica gel, ethyl acetate/hexane=1/2). The objective fraction was concentrated under reduced pressure, ethanol was added to the residue, and the precipitates produced were collected by filtration. The precipitates were washed with ethanol and dried under reduced pressure to give the title compound (1.2g, 3.4mmol). ¹H-NMR (DMSO-d₆)δ: 2.86 (2H, t, J=8.0Hz), 3.41 (2H, t, J=8.0Hz), 4.59 (2H, s), 7.24-7.33 (6H, m), 7.42 (1H, dd, J=9.2, 2.2Hz), 8.12 (1H, d, J=2.2Hz).
Mass (ESI+); 354 (M+H), 356

### Synthesis Example 2: 1-benzyl-6-(4-methylphenyl)-2,3-dihydro-1H-pyrrolo [2,3-b] quinoline-4-ylamine

1-benzyl-6-bromo-2,3-dihydro-1H-pyrrolo [2,3-b] quinoline-4-ylamine (70mg, 0.2mmol) was suspended in 0.5ml of toluene, and Pd(Ph₃P)₄ (6mg), 2M aqueous sodium carbonate solution (0.2ml) and a solution of 4-methylphenyl boronate (30mg) in ethanol (0.25ml) were added thereto for reaction at 90°C for 16 hours. Water and ethyl acetate were added to the reaction mixture. After separation, the organic layer was washed with water, dried over anhydrous sodium sulfate and concentrated under reduced pressure. The resultant residue was then subjected to column chromatography on silica gel (2g of silica gel, ethyl acetate/hexane=1/2). The objective fraction was concentrated and hydrochloric acid was added to the residue in the presence of ethanol. The precipitates were collected by filtration. The precipitates were washed with ethanol and then dried under reduced pressure to give the title compound (15mg).
¹H-NMR (CDCl₃)δ: 2.41(3H, s), 2.92 (2H, t, J=8.0Hz), 3.50 (2H, t, J=8.0Hz), 4.75 (2H, s), 7.24-7.38 (7H, m), 7.57 (2H, d), 7.69 (3H, m).
Mass (ESI+); 366 (M+H)

### Synthesis Example 3: 1-benzyl-6-(3-thienyl)-2,3-dihydro-1H-pyrrolo [2,3-b] quinoline-4-ylamine hydrochloride

1-benzyl-6-bromo-2,3-dihydro-1H-pyrrolo [2,3-b] quinoline-4-ylamine (140mg, 0.4mmol) was suspended in 1ml of toluene, and Pd(Ph₃P)₄ (12mg), 2M aqueous sodium carbonate solution (0.4ml) and a solution of 3-thiophene boronate (56mg) in ethanol (0.5ml) were added thereto for reaction at 90°C for 16 hours. Water and ethyl acetate were added to the reaction solution. After separation, the organic layer was washed with water, dried over anhydrous sodium sulfate and concentrated under reduced pressure. The residue was then subjected to column chromatography on silica gel (2g of silica gel, ethyl acetate/hexane=1/2). The objective fraction was concentrated and hydrochloric acid was added to the residue in the presence of ethanol. The precipitates were collected by filtration. The precipitates were washed with ethanol and then dried under reduced pressure to give the title compound (52mg).
¹H-NMR (DMSO-d₆)δ: 3.01(2H, t, J=8.0Hz), 3.73 (2H, t, J=8.0Hz), 4.92 (2H, s), 7.40 (5H, bs), 7.70-7.88 (3H, m), 8.02-8.09 (2H, m), 8.54 (1H, s). Mass (ESI+); 358 (M+H)

### Synthesis Example 4: N-benzyl-1-benzyl-6-bromo-2,3-dihydro-1H-pyrrolo [2,3-b] quinoline-4-ylamine

1-benzyl-6-bromo-2,3-dihydro-1H-pyrrolo [2,3-b] quinoline-4-ylamine (70mg, 0.2mmol) was dissolved in 1ml of dimethylformamide, and 2-tert-butylimino-2-diethylamino-1,3-dimethylperhydro-1,3,2-diazaphospholine (0.24ml) and benzyl bromide (0.095ml) were added thereto for reaction at 80°C for 1 hour. Water and ethyl acetate were added to the reaction mixture. After separation, the organic layer was washed with water, dried over anhydrous sodium sulfate and then concentrated under reduced pressure. The residue was purified by preparative HPLC (YMC CombiPrep ODS, 20x50mm). The objective fraction was dried under reduced pressure to give the title compound (44mg).
¹H-NMR (CDCl₃)δ: 3.13(2H, t, J=8.0Hz), 3.54 (2H, t, J=8.0Hz), 4.72 (2H, s), 4.82 (2H, s), 7.22-7.37 (10H, m), 7.50 (1H, dd, J=9.0, 2.0Hz), 7.69 (1H, d, J=9.0Hz), 8.00 (1H, d, J=2.0Hz).
Mass (ESI+); 444 (M+H), 446

### Synthesis Example 5: N,N-dibenzyl-1-benzyl-6-bromo-2,3-dihydro-1H-pyrrolo [2,3-b] quinoline-4-ylamine

1-benzyl-6-bromo-2,3-dihydro-1H-pyrrolo [2,3-b] quinoline-4-ylamine (70mg, 0.2mmol) was dissolved in 1ml of dimethylformamide, and 2-tert-butylimino-2-diethylamino-1,3-dimethylperhydro-1,3,2-diazaphospholine (0.24ml) and benzyl bromide (0.095ml) were added thereto for reaction at 80°C for 1 hour. Water and ethyl acetate were added to the reaction solution. After separation, the organic layer was washed with water, dried over anhydrous sodium sulfate and then concentrated under reduced pressure. The residue was purified by preparative HPLC (YMC CombiPrep ODS, 20×50mm). The objective fraction was dried under reduced pressure to give the title compound (32mg).
¹H-NMR (CDCl₃)δ: 2.61(2H, t, J=8.0Hz), 3.49 (2H, t, J=8.0Hz), 4.27 (4H, s), 4.99 (2H, s), 7.17-7.38 (15H, m), 7.74 (1H, dd, J=8.8, 2.0Hz), 7.97 (1H, d, J=8.8Hz), 8.16 (1H, d, J=2.0Hz).
Mass (ESI+); 534 (M+H), 536

### Synthesis Example 6: N-allyl-1-benzyl-6-bromo-2,3-dihydro-1H-pyrrolo [2,3-b] quinoline-4-ylamine

1-benzyl-6-bromo-2,3-dihydro-1H-pyrrolo [2,3-b] quinoline-4-ylamine (70mg, 0.2mmol) was dissolved in 1ml of dimethylformamide, and 2-tert-butylimino-2-diethylamino-1,3-dimethylperhydro-1,3,2-diazaphospholine (0.24ml) and allyl bromide (0.07ml) were added thereto for reaction at 80°C for 1 hour. Water and ethyl acetate were added to the reaction solution. After separation, the organic layer was washed with water, dried over anhydrous sodium sulfate and then concentrated under reduced pressure. The residue was purified by preparative HPLC (YMC CombiPrep ODS, 20x50mm). The objective fraction was dried under reduced pressure to give the title compound (26mg).
¹H-NMR (CDCl₃)δ: 3.23(2H, t, J=8.0Hz), 3.62 (2H, t, J=8.0Hz), 4.07 (2H, bs), 4.83 (2H, s), 5.12-5.24 (2H, m), 5.91-6.00 (1H, m), 7.35 (5H, bs), 7.44 (1H, dd, J=8.8, 2.0Hz), 7.61 (1H, d, J=8.8Hz), 8.03 (1H, d, J=2.0Hz).
Mass (ESI+); 394 (M+H), 396

### Synthesis Example 7: N,N-diallyl-1-benzyl-6-bromo-2,3-dihydro-1H-pyrrolo[2,3-b] quinoline-4-ylamine

1-benzyl-6-bromo-2,3-dihydro-1H-pyrrolo[2,3-b] quinoline-4-ylamine (70mg, 0.2mmol) was dissolved in 1ml of dimethylformamide, and 2-tert-butylimino-2-diethylamino-1,3-dimethylperhydro-1,3,2-diazaphospholine (0.24ml) and allyl bromide (0.07ml) were added thereto for reaction at 80°C for 1 hour. Water and ethyl acetate were added to the reaction solution. After separation, the organic layer was washed with water, dried over anhydrous sodium sulfate and then concentrated under reduced pressure. The residue was purified by preparative HPLC (YMC CombiPrep ODS, 20x50mm). The objective fraction was dried under reduced pressure to give the title compound (12mg).
¹H-NMR (CDCl₃)δ: 3.20 (2H, t, J=8.0Hz), 3.70 (2H, t, J=8.0Hz), 3.88 (4H, d), 5.01 (2H, s), 5.20-5.29 (4H, m), 5.68-5.89 (2H, m), 7.36 (5H, bs), 7.67 (1H, dd, J=9.0, 2.0Hz), 7.90 (1H, d, J=9.0Hz), 7.97 (1H, d, J=2.0Hz).
Mass (ESI+); 434 (M+H), 436

### Synthesis Example 8: N-methyl-1-benzyl-6-bromo-2,3-dihydro-1H-pyrrolo [2,3-b] quinoline-4-ylamine

1-benzyl-6-bromo-2,3-dihydro-1H-pyrrolo [2,3-b] quinoline-4-ylamine (50mg) was dissolved in 0.5ml of dimethylformamide, and di-isopropyl ethylamine (0.05ml) and methyl iodide (0.5ml) were added thereto for reaction at room temperature for 40 hours. The reaction solution was concentrated under reduced pressure. The residue was then purified by preparative HPLC (YMC CombiPrep ODS, 20x50mm). The objective fraction was dried under reduced pressure to give the title compound (8mg).
¹H-NMR (CDCl₃)δ: 3.19 (3H, s), 3.39 (2H, t, J=8.0Hz), 3.66 (2H, t, J=8.0Hz), 4.81 (2H, s), 7.28-7.57 (7H, m), 7.97 (1H, s).
Mass (ESI+); 368 (M+H), 370

### Synthesis Example 9: N,N-dimethyl-1-benzyl-6-bromo-2,3-dihydro-1H-pyrrolo [2,3-b] quinoline-4-ylamine

1-benzyl-6-bromo-2,3-dihydro-1H-pyrrolo [2,3-b] quinoline-4-ylamine (50mg) was dissolved in 0.5ml of dimethylformamide, and di-isopropyl ethylamine (0.05ml) and methyl iodide (0.5ml) were added thereto for reaction at room temperature for 40 hours. The reaction solution was concentrated under reduced pressure. The residue was then purified by preparative HPLC (YMC CombiPrep ODS, 20x50mm). The objective fraction was dried under reduced pressure to give the title compound (5mg).
¹H-NMR (CDCl₃)δ: 3.14 (6H, s) , 3.37 (2H, t, J=8.0Hz), 3.74 (2H, t, J=8.0Hz), 4.94 (2H, s), 7.38 (5H, bs), 7.67 (1H, dd, J=9.0, 2.0Hz), 7.77 (1H, d, J=9.0Hz), 7.95 (1H, d, J=2.0Hz). Mass (ESI+); 382 (M+H), 384

### Synthesis Example 10: 6-bromo-1-(4-fluorobenzyl)-2,3-dihydro-1H-pyrrolo [2,3-b] quinoline-4-ylamine

2-Pyrrolidone (0.76ml) was added to a suspension of sodium hydride (60% mineral oil suspension) (440mg) in N,N-dimethylformamide (10ml) and the mixture was stirred at room temperature for 15 minutes, and 4-fluorobenzyl bromide (1.37ml) was added thereto. The reaction mixture was stirred at room temperature for 15 hours. Water was added to the mixture and the mixture was extracted with diethyl ether. The extract solution was washed with brine, dried over anhydrous magnesium sulfate and then concentrated under reduced pressure. The resultant residue was purified by chromatography on silica gel (n-hexane/ethylacetate=1/2) to give 1-(4-fluorobenzyl)-2-pyrrolidone (1.28g).

Phosphorus oxychloride (0.30ml) was added to a solution of 1-(4-fluorobenzyl)-2-pyrrolidone (600mg) in chloroform (3ml) and the mixture was stirred at room temperature for 30 minutes, and then 2-amino-5-bromobenzonitrile (583mg) was added thereto. The reaction mixture was refluxed for 3 hours while heating. Ice-water was added to the mixture. Further, the mixture was neutralized by adding 20% aqueous sodium hydroxide solution and then extracted with chloroform. The extract was washed with brine, dried over anhydrous magnesium sulfate and then concentrated under reduced pressure to give 5-bromo-2-{(1-(4-fluorobenzyl)-2-pyrrolidinylidene) amino} benzonitrile (1.01g).

Under nitrogen atmosphere, a solution of 5-bromo-2-{(1-(4-fluorobenzyl)-2-pyrrolidinylidene) amino} benzonitrile (1.01g) in tetrahydrofuran (8ml) was cooled to -40°C. Lithium di-isopropyl amide (2.0M heptane/tetrahydrofuran/ethylbenzene solution: 1.63ml) was added dropwise to the solution while stirring at -40°C. The reaction solution was gradually heated to room temperature and stirred for 1 hour. Ice-water was added to the reaction solution and the solution was extracted with ethyl acetate. The extract was washed with brine, dried over anhydrous magnesium sulfate and then concentrated under reduced pressure. The resultant residue was purified by chromatography on silica gel (n-hexane/ethyl acetate=2/1) to give the title compound (347mg).
¹H-NMR (DMSO-d₆) δ: 2.86 (2H, t, J=8.0Hz), 3.42 (2H, t, J=8.0Hz), 4.58 (2H, s), 7.14 (2H, d, J=8.8Hz), 7.29-7.46 (4H, m), 8.13 (1H, d, J=2.2Hz)
Mass (APCI+); 372 (M+H), 374

### Synthesis Example 11: 6-bromo-1- (2-phenethyl)-2,3-dihydro-1H-pyrrolo [2,3-b] quinoline-4-ylamine hydrochloride

2-Pyrrolidone (0.76ml) was added to a suspension of sodium hydride (60% mineral oil suspension) (440mg) in N,N-dimethylformamide (10ml). The reaction mixture was stirred at room temperature for 15 minutes, and (2-bromoethyl) benzene (1.50ml) was added thereto. The reaction mixture was stirred at room temperature for 15 hours. Water was added to the mixture and the mixture was extracted with diethyl ether. The extract was washed with brine, dried over anhydrous magnesium sulfate and then concentrated under reduced pressure. The resultant residue was purified by chromatography on silica gel (n-hexane/ethylacetate=1/2) to give 1-(2-phenethyl)-2-pyrrolidone (0.438g).

Phosphorus oxychloride (0.23ml) wa added to a solution of 1-(2-phenethyl)-2-pyrrolidone (438mg) in chloroform (3ml). The mixture was stirred at room temperature for 30 minutes, and then 2-amino-5-bromobenzonitrile (435mg) was added thereto. The reaction mixture was refluxed for 3 hours while heating and ice-water was added thereto. Further, the mixture was neutralized by adding 20% aqueous sodium hydroxide solution and then extracted with chloroform. The extract was washed with brine, dried over anhydrous magnesium sulfate and then concentrated under reduced pressure to give 5-bromo-2-{(1-(2-phenethyl)-2-pyrrolidinylidene) amino} benzonitrile (736mg).

Under nitrogen atmosphere, a solution of 5-bromo-2-{(1-(2-phenethyl)-2-pyrrolidinylidene) amino} benzonitrile (736mg) in tetrahydrofuran (6ml) was cooled to -40°C. Lithium di-isopropyl amide (2.0M heptane/tetrahydrofuran/ethylbenzene solution: 1.20ml) was added dropwise to the solution while stirring at -40°C. The reaction solution was gradually heated to room temperature and stirred for 1 hour. Ice-water was added the reaction solution and the mixture was extracted with ethyl acetate. The extract solution was washed with brine, dried over anhydrous magnesium sulfate and concentrated under reduced pressure. The resultant residue was purified by chromatography on silica gel (n-hexane/ethyl acetate=2/1) and treated with 4N HCl-ethyl acetate to give the title compound (254mg).
¹H-NMR (DMSO-d₆)δ: 2.80-3.00 (2H, m), 3.15-3.30 (2H, m), 3.83 (2H, t, J=8.0Hz), 3.93 (2H, t, J=8.0Hz), 7.15-7.45 (5H, m), 7.75-7.90 (2H, m), 8.39 (1H, s)

### Synthesis Example 12: 6-bromo-1-(3-pyridinylmethyl)-2,3-dihydro-1H-pyrrolo [2,3-b] quinoline-4-ylamine dihydrochloride

2-Pyrrolidone (0.76ml) was added to a suspension of sodium hydride (60% mineral oil suspension) (880mg) in N,N-dimethylformamide (10ml). The mixture was stirred at room temperature for 15 minutes, and 3-(chloromethyl)pyridine hydrochloride (1.80g) was added thereto. The reaction mixture was stirred at room temperature for 15 hours. Water was added to the mixture and the mixture was extracted with dichloroform. The extract was washed with brine, dried over anhydrous magnesium sulfate and concentrated under reduced pressure. The resultant residue was treated with 4N HCl-ethyl acetate to give 1-(3-pyridinylmethyl)-2-pyrrolidone hydrochloride (2.02g).

Phosphorus oxychloride (0.31ml) was added to a solution of 1-(3-pyridinylmethyl)-2-pyrrolidone hydrochloride (600mg) in chloroform (3ml) and the mixture was stirred at room temperature for 30 minutes, and then 2-amino-5-bromobenzonitrile (530mg) was added thereto. The reaction mixture was refluxed for 3 hours while heating and then ice-water was added thereto. Further, the mixture was neutralized by adding 20% aqueous sodium hydroxide solution and extracted with chloroform. The extract was washed with brine, dried over anhydrous magnesium sulfate and concentrated under reduced pressure to give 5-bromo-2-{(1-(3-pyridinylmethyl)-2-pyrrolidinylidene) amino}benzonitrile (736mg).

Under nitrogen atmosphere, a solution of 5-bromo-2-{(1-(3-pyridinylmethyl)-2-pyrrolidinylidene) amino} benzonitrile (940mg) in tetrahydrofuran (10ml) was cooled to -40°C. Lithium di-isopropyl amide (2.0M heptane/tetrahydrofuran/ethylbenzene solution: 1.99ml) was added dropwise to the solution while stirring at -40°C. The reaction solution was gradually heated to room temperature and stirred for 1 hour. Ice-water was added to the reaction mixture and the mixture was extracted with ethyl acetate. The extract was washed with brine, dried over anhydrous magnesium sulfate and concentrated under reduced pressure. The resultant residue was purified by chromatography on silica gel (n-hexane/ethyl acetate=2/1) and treated with 4N HCl-ethyl acetate to give the title compound (198mg).
¹H-NMR (DMSO-d₆)δ: 3.00 (2H, t, J=8.0Hz), 3.79 (2H, t, J=8.0Hz), 5.28 (2H,s), 7.56 (2H, m), 7.83 (1H, d, J=8.0Hz), 7.90-8.10 (2H, m), 8.46 (1H, s), 8.54 (1H, d, J=8.0Hz), 8.85 (1H, d, J=5.8Hz), 9.05 (1H, s)
Mass (APCI+); 355 (M+H), 357

### Synthesis Example 13: 1-benzyl-6-fluoro-2,3-dihydro-1H-pyrrolo [2,3-b] quinoline-4-ylamine hydrochloride

Phosphorus oxychloride (0.51ml) was added to a solution of 1-benzyl-2-pyrrolidone (0.84ml) in chloroform (3ml). The solution was stirred at room temperature for 30 minutes, and 2-amino-5-fluorobenzonitrile (0.65ml) was added thereto. The reaction mixture was refluxed for 3 hours while heating and then ice-water was added thereto. Further, the mixture was neutralized by adding 20% aqueous sodium hydroxide solution and extracted with chloroform. The extract was washed with brine, dried over anhydrous magnesium sulfate and then concentrated under reduced pressure to give 5-fluoro-2-{(1-benzyl-2-pyrrolidinylidene) amino} benzonitrile (1.68g).

A solution of 5-fluoro-2-{(1-benzyl-2-pyrrolidinylidene) amino} benzonitrile (500mg) in tetrahydrofuran (2ml) was added to a solution of sodium hexamethyldisilazane (3.91ml) in tetrahydrofuran (3ml) cooled to -78°C. After stirring at the same temperature for 15 minutes, the reaction solution was gradually heated to -20°C and stirred for 2 hours. Further, the reaction solution was heated to 40°C, stirred for 15 hours. Brine was added to the reaction solution and the reaction solution was extracted with ethyl acetate. The extract solution was washed with brine, dried over anhydrous magnesium sulfate and concentrated under reduced pressure. The resultant residue was purified by chromatography on silica gel (n-hexane/ethyl acetate=2/1) and treated with 4N HCl-ethyl acetate to give the title compound (60mg).
¹H-NMR (DMSO-d₆)δ: 2.99 (2H, t, J=8.0Hz), 3.72 (2H, t, J=8.0Hz), 4.95 (2H,s), 7.25-7.65 (6H, m), 7.95-8.15 (2H, m) Mass (APCI+); 294 (M+H)

### Synthesis Example 14: 1-benzyl-7-bromo-1,2,3,4-tetrahydrobenzo [b] [1,8] naphthyridine-5-ylamine hydrochloride

2-Piperidone (991mg) was added to a suspension of sodium hydride(60% mineral oil suspension) (440mg) in N,N-dimethylformamide (10ml). The reaction mixture was stirred at room temperature for 15 minutes, and benzylbromide (1.31ml) was added thereto. The reaction mixture was stirred at room temperature for 4 hours. Water was added to the reaction mixture and the reaction mixture was extracted with diethyl ether. The extract was washed with brine, dried over anhydrous magnesium sulfate and concentrated under reduced pressure. The resultant residue was purified by chromatography on silica gel (n-hexane/ethyl acetate=1/1) to give 1-benzyl-2-piperidone (1.30g).

Phosphorus oxychloride (0.29ml) was added to a solution of 1-benzyl-2-piperidone (567mg) in chloroform (3ml). The reaction mixture was refluxed for 3 hours while heating and ice-water was added thereto. Further, the mixture was neutralized by adding 20% aqueous sodium hydroxide solution and extracted with chloroform. The extract was washed with brine, dried over anhydrous magnesium sulfate and concentrated under reduced pressure to give 5-bromo-2-{(1-benzyl-2-piperidinylidene) amino} benzonitrile (1.05g).

5-bromo-2-{(1-benzyl-2-piperidinylidene)amino} benzonitrile (1.01g) was dissolved in nitrobenzene (5ml), and zinc chloride (466mg) was added to the solution which was then stirred at 155°C for 1 hour. After cooling, the pH of the reaction mixture solution was adjusted to 10 by adding 20% aqueous sodium hydroxide solution, and then extracted with chloroform. The extract was washed with brine, dried over anhydrous magnesium sulfate and then concentrated under reduced pressure. The resultant residue was purified by chromatography on silica gel (n-hexane/ethyl acetate=9/1→3/2) and treated with 4N HCl-ethyl acetate to give the title compound (551mg).
¹H-NMR (DMSO-d₆)δ: 1.85-2.00 (2H, m), 2.59 (2H, t, J=6.0Hz), 3.10-3.50 (2H, m), 5.12 (2H,s), 7.25-7.45 (5H, m), 7.80 (1H, dd, J=1.8Hz, 8.8Hz), 8.03 (1H, d, J=8.8Hz), 8.54 (1H, d, J=1.8Hz)
Mass (APCI+); 368 (M+H), 370

### Synthesis Example 15: 1-benzyl-7-bromo-2,2,4,5-tetrahydro-1H-azepino [2,3-b] quinoline-6-ylamine hydrochloride

ε-caprolactam (1.13g) was added to a suspension of sodium hydride (60% mineral oil suspension) (440mg) in N,N-dimethylformamide (10ml). The reaction mixture was stirred at room temperature for 15 minutes, and benzylbromide (1.31ml) was added thereto. The reaction mixture was stirred at room temperature for 4 hours. Water was added to the mixture and the mixture was extracted with diethyl ether. The extract was washed with brine, dried over anhydrous magnesium sulfate and concentrated under reduced pressure. The resultant residue was purified by chromatography on silica gel (n-hexane/ethyl acetate=1/1) to give N-benzyl- ε-caprolactam (1.72g).

Phosphorus oxychloride (0.29ml) was added to a solution of N-benzyl-ε-caprolactam (607mg) in chloroform (3ml). The reaction mixture was refluxed for 3 hours while heating and ice-water was added thereto. Further, the mixture was neutralized by adding 20% aqueous sodium hydroxide solution and then extracted with chloroform. The extract was washed with brine, dried over anhydrous magnesium sulfate and concentrated under reduced pressure to give 1-benzyl-2-{(4-bromo-2-cyanophenyl)imino} hexahydro-1H-azepine (1.08g).

1-benzyl-2-{(4-bromo-2-cyanophenyl) imino}-hexahydro-1H-azepine (1.08g) was dissolved in nitrobenzene (5ml), and zinc chloride (463mg) was added to the solution which was then stirred at 155°C for 1 hour. After cooling, the pH of the solution was adjusted to 10 by adding ammonia solution, and then extracted with chloroform. The extract was washed with brine, dried over anhydrous magnesium sulfate and concentrated under reduced pressure. The resultant residue was purified by chromatography on silica gel (n-hexane/ethyl acetate=9/1→3/2) and treated with 4N HCl-ethyl acetate to give the title compound (475mg).
¹H-NMR (DMSO-d₆)δ: 1.55-1.85 (4H, m), 2.71 (2H, m), 3.51 (2H, m),4.94 (2H,s), 7.30-7.50 (5H, m), 7.84 (1H, dd, J=2.0Hz, 8.8Hz), 8.07 (1H, d, J=8.8Hz), 8.60 (1H, d, J=2.0Hz)
Mass (APCI+); 382 (M+H), 384

### Synthesis Example 16: 1-benzyl-6-(benzofuran-2-yl)-2,3-dihydro-1H- pyrrolo [2,3-b] quinoline-4-ylamine hydrochloride

1-benzyl-6-bromo-2,3-dihydro-1H-pyrrolo [2,3-b] quinoline-4-ylamine (140mg, 0.4mmol) was suspended in 1 ml of toluene, and Pd(Ph₃P)₄ (12mg), 2M aqueous sodium carbonate solution (0.4ml) and a solution of benzofuran-2-ylboronate (72mg) in ethanol (0.5ml) were added to the mixture for reaction at 90°C for 16 hours. Water and ethyl acetate were added to the reaction solution. After separation, the organic layer was washed with water, dried over anhydrous sodium sulfate and concentrated under reduced pressure. The resultant residue was then subjected to column chromatography on silica gel (2g of silica gel, ethyl acetate/hexane=1/2). The objective fraction was concentrated and hydrochloric acid was added to the residue in the presence of ethanol. The precipitates produced were collected by filtration, washed with ethanol and then dried under reduced pressure to give the title compound (63mg). ¹H-NMR (DMSO-d₆)δ: 3.01(2H, t, J=8.0Hz), 3.74 (2H, t, J=8.0Hz), 4.95 (2H, s), 7.26-7.74 (12H, m), 7.97 (1H, d, J=8.4Hz), 8.19 (1H, d, J=8.4Hz), 8.75 (1H, s).
Mass (ESI+); 392 (M+H)

### Synthesis Example 17: 1-benzyl-6-(3-acetaminophenyl)-2,3-dihydro-1H- pyrrolo [2,3-b] quinoline-4-ylamine hydrochloride

1-benzyl-6-bromo-2,3-dihydro-1H-pyrrolo [2,3-b] quinoline-4-ylamine (140mg, 0.4mmol) was suspended in 1 ml of toluene, and Pd(Ph₃P)₄ (12mg), 2M aqueous sodium carbonate solution (0.4ml) and a solution of 3-acetaminophenyl boronate (79mg) in ethanol (0.5ml) were added to the mixture for reaction at 90°C for 16 hours. Water and ethyl acetate were added to the reaction mixture. After separation, the organic layer was washed with water, dried over anhydrous sodium sulfate and concentrated under reduced pressure. The residue was then subjected to column chromatography on silica gel (2g of silica gel, ethyl acetate/hexane=1/2). The objective fraction was concentrated and hydrochloric acid was added to the residue in the presence of ethanol. The precipitates produced were collected by filtration, washed with ethanol and then dried under reduced pressure to give the title compound (17mg).
¹H-NMR (DMSO-d₆)δ: 2.09 (3H, s) , 3.00 (2H, t, J=8.0Hz), 3.73 (2H, t, J=8.0Hz), 4.98 (2H, s), 7.35-7.64 (9H, m), 7.88 (1H, d, J=8.4Hz), 7.99 (2H, bs), 8.01 (1H, d, J=8.4Hz), 8.44 (1H, s) .
Mass (ESI+); 409 (M+H)

### Synthesis Example 18: 1-(4-tert-butylbenzyl)-6-bromo-2,3-dihydro-1H- pyrrolo [2,3-b] quinoline-4-ylamine hydrochloride

1-benzyl-6-bromo-2,3-dihydro-1H-pyrrolo [2,3-b] quinoline-4-ylamine (350mg, 1mmol) was suspended in 2 ml of dichloromethane and BBr₃ (1.4ml) was added dropwise thereto. The suspension was stirred at room temperature for 8 hours, ice-water was added thereto. The reaction mixture was alkalized by adding 30% aqueous sodium hydroxide solution, and extracted with dichloromethane containing ethanol. After concentration under reduce pressure, the residue was crystallized from dichloromethane to give 50mg of 6-bromo-2,3-dihydro-1H-pyrrolo [2,3-b] quinoline-4-ylamine, which was then dissolved in N,N-dimethylformamide (1ml). 2-tert-butylimino-2-diethylamino-1,3-dimethylperhydro-1,3,2-diazaphosphorine (0.088ml) and 4-tert-butyl benzyl bromide (0.060ml) was added to the solution and the solution was left for reaction at room temperature for 40 hours. Ice-water and dichloromethane were added to the reaction mixture. After separation, the organic layer was washed with water, dried over anhydrous sodium sulfate and then concentrated under reduced pressure. The residue was then subjected to column chromatography on silica gel (2g of silica gel, ethyl acetate/hexane=1/2). The objective fraction was concentrated and hydrochloric acid was added to the residue in the presence of ethanol. The precipitates produced were collected by filtration, washed with ethanol and then dried under reduced pressure to give the title compound (20mg).
¹H-NMR (DMSO-d₆)δ: 1.27 (9H, s), 2.98 (2H, t, J=8.0Hz), 3.73 (2H, t, J=8.0Hz), 4.87 (2H, s), 7.29 (1H, d, J=8.2Hz), 7.42 (1H, d, J=8.2Hz), 7.48 (2H, bs), 7.81 (2H, s), 8.43 (1H, s). Mass (ESI+); 410 (M+H)

### Synthesis Example 19: 1-(4-cyanobenzyl)-6-bromo-2,3-dihydro-1H- pyrrolo [2,3-b] quinoline-4-ylamine hydrochloride

1-benzyl-6-bromo-2,3-dihydro-1H-pyrrolo [2,3-b] quinoline-4-ylamine (350mg, 1mmol) was suspended in 2 ml of dichloromethane and BBr₃ (1.4ml) was added dropwise thereto. The suspension was stirred at room temperature for 8 hours and ice-water was added thereto. The mixture was alkalized by adding 30% aqueous sodium hydroxide solution, and extracted with dichloromethane containing ethanol. After concentration under reduce pressure, the resultant residue was crystallized from dichloromethane to give 50mg of 6-bromo-2,3-dihydro-1H-pyrrolo [2,3-b] quinoline-4-ylamine, which was then dissolved in N,N-dimethylformamide (1ml). 2-tert-butylimino-2-diethylamino-1,3-dimethylperhydro-1,3,2-diazaphosphorine (0.088ml) and 4-cyanobenzyl bromide (66mg) were added to the solution and the solution was left for reaction at room temperature for 40 hours. Ice-water and dichloromethane were added to the reaction mixture, and after separation, the organic layer was washed with water, dried over anhydrous sodium sulfate and then concentrated under reduced pressure. The residue was then subjected to column chromatography on silica gel (2g of silica gel, ethyl acetate/hexane=1/2). The objective fraction was concentrated and hydrochloric acid was added to the residue in the presence of ethanol. The precipitates produced were collected by filtration, washed with ethanol and then dried under reduced pressure to give the title compound (20mg).
¹H-NMR (DMSO-d₆)δ: 3.00 (2H, t, J=8.0Hz), 3.74 (2H, t, J=8.0Hz), 5.06 (2H, s), 7.50-7.62 (4H, m), 7.82-7.90 (6H, m), 8.45 (1H, s).
Mass (ESI+); 379 (M+H)

### Synthesis Example 20: 1-(3,5-dimethoxybenzyl)-6-bromo-2,3-dihydro-1H- pyrrolo [2,3-b] quinoline-4-ylamine hydrochloride

1-benzyl-6-bromo-2,3-dihydro-1H-pyrrolo [2,3-b] quinoline-4-ylamine (350mg, 1mmol) was suspended in 2 ml of dichloromethane and BBr₃ (1.4ml) was added dropwise thereto. The suspension was stirred at room temperature for 8 hours, and ice-water was added thereto. The mixture was alkalized by adding 30% aqueous sodium hydroxide solution, and extracted with dichloromethane containing ethanol. After concentration under reduce pressure, the resultant residue was crystallized from dichloromethane to give 50mg of 6-bromo-2,3-dihydro-1H-pyrrolo [2,3-b] quinoline-4-ylamine, which was then dissolved in N,N-dimethylformamide (1ml). 2-tert-butylimino-2-diethylamino-1,3-dimethylperhydro-1,3,2-diazaphosphorine (0.088ml) and 3,5-dimethoxybenzyl bromide (75mg) was added to the solution, and the solution was left for reaction at room temperature for 40 hours. Ice-water and dichloromethane were added to the reaction mixture, and after separation, the organic layer was washed with water, dried over anhydrous sodium sulfate and then concentrated under reduced pressure. The resultant residue was subjected to column chromatography on silica gel (2g of silica gel, ethyl acetate/hexane=1/2). The objective fraction was concentrated and then hydrochloric acid was added to the residue in the presence of ethanol. The precipitates produced were collected by filtration, washed with ethanol and then dried under reduced pressure to give the title compound (17mg).
¹H-NMR (DMSO-d₆)δ: 2.98 (2H, t, J=8.0Hz), 3.70-3.74 (8H, m), 6.42-6.54 (3H, m), 7.48 (2H, bs), 7.80 (2H, s), 8.43 (1H, s) .
Mass (ESI+); 414 (M+H)

### Synthesis Example 21: 1-(4-methoxybenzyl)-6-bromo-2,3-dihydro-1H- pyrrolo [2,3-b] quinoline-4-ylamine hydrochloride

1-benzyl-6-bromo-2,3-dihydro-1H-pyrrolo [2,3-b] quinoline-4-ylamine (350mg, 1mmol) was suspended in 2 ml of dichloromethane and BBr₃ (1.4ml) was added dropwise thereto. The suspension was stirred at room temperature for 8 hours, and ice-water was added thereto. The mixture was alkalized by adding 30% aqueous sodium hydroxide solution, and extracted with dichloromethane containing ethanol. After concentration under reduce pressure, the resultant residue was crystallized from dichloromethane to give 50mg of 6-bromo-2,3-dihydro-1H-pyrrolo [2,3-b] quinoline-4-ylamine, which was then dissolved in N,N-dimethylformamide (1ml), 2-tert-butylimino-2-diethylamino-1,3-dimethylperhydro-1,3,2-diazaphosphorine (0.088ml) and 4-methoxybenzyl bromide (0.045ml) were added to the solution, and the solution was left for reaction at room temperature for 40 hours. Ice-water and dichloromethane were added to the reaction mixture , and after separation, the organic layer was washed with water, dried over anhydrous sodium sulfate and then concentrated under reduced pressure. The residue was then subjected to column chromatography on silica gel (2g of silica gel, ethyl acetate/hexane=1/2). The objective fraction was concentrated and then hydrochloric acid was added to the residue in the presence of ethanol. The precipitates produced were collected by filtration, washed with ethanol and then dried under reduced pressure to give the title compound (17mg).
¹H-NMR (DMSO-d₆)δ: 2.95 (2H, t, J=8.0Hz), 3.69 (2H, t, J=8.0Hz), 3.75 (3H, s), 4.85 (2H, s), 6.95 (2H, d, J=8.8Hz), 7.33 (2H, d, J=8.8Hz), 7.45 (2H, bs), 7.84 (2H, bs), 8.43 (1H, s).
Mass (ESI+); 384 (M+H)

Vasoactive drugs (including drugs for preventing/treating myocardial infarction, heart failure, etc.) which contain, as an active agent, any of compounds having GPR 14-antagonizing activity or salts thereof according to the present invention can be prepared according to, for example, the following procedures.

### Formulation Examples

1. Capsule formulation

| | |
|---|---|
| (1) Compounds obtained in Example 1 | 40mg |
| (2) Lactose | 70mg |
| (3) Microcrystalline cellulose | 9mg |
| (4) Magnesium stearate | 1mg |
| (1 capsule unit=120mg) | |

(1), (2), (3) and 1/2 of (4) are mixed together and granulated. Then, the rest of (4) is added to the granule and the mixture is encapsulated into gelatin capsules.
2. Tablet formulation

| | |
|---|---|
| (1) Compounds obtained in Example 1 | 40mg |
| (2) Lactose | 58mg |
| (3) Corn starch | 18mg |
| (4) Microcrystalline cellulose | 3.5mg |
| (5) Magnesium stearate | 0.5mg |
| (1 tablet unit=120mg) | |

(1), (2), (3), 2/3 of (4) and 1/2 of (5) are mixed together and granulated. Then, the rest of (4) and (5) are added to the granule and the mixture is formulated into tablets by compression molding.

### Industrial Applicability

Since the inventive compounds having GPR 14-antagonizing activity [including compounds represented by formula (I), (II) or (II')] or salts thereof have potent GPR14 antagonistic activity, they can advantageously be used in various vasoactive drugs (preferably vasoconstriction inhibitor) as well as in treatment of various diseases (preferably ischemic myocardial infarction or congestive heart failure).

## Claims

1. A vasoactive agent which comprises a compound having GPR 14 antagonistic action or salts thereof.

2. The agent according to claim 1, which is a vasoconstriction inhibitor.

3. The agent according to claim 1, which is the agent for preventing and/or treating hypertension, arteriosclerosis, hypercardia, myocardial infarction or heart failure.

4. A GPR 14 antagonistic agent which comprises a non-peptide compound or salts thereof.

5. A GPR 14 antagonistic agent which comprises a quinoline derivative or salts thereof.

6. A GPR 14 antagonistic agent which comprises 4-amino quinoline derivative or salts thereof.

7. A GPR 14 antagonistic agent which comprises a compound having the formula (I) : 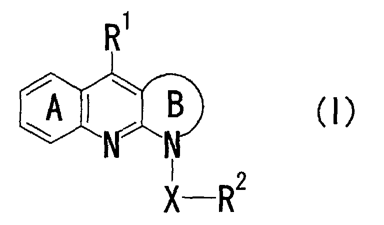 wherein A is a benzene ring which may be substituted, B is a 5- to 8-membered ring which may be substituted , X is a divalent group containing 1 to 4 atoms in its linear chain, R¹ is an amino group which may be substituted, and R² is a cyclic group which may be substituted; or salts thereof.

8. The agent according to claim 7, wherein A is a benzene ring which may be. substituted by: (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl, (16') C₁₋₄ alkylsulfinyl; (3) a nitro group; (4) a halogen atom; (5) an amino group which may be substituted by (1') alkyl group which may be substituted, (2') cycloalkyl group which may be substituted, (3') alkenyl group which may be substituted, (4') cycloalkenyl group which may be substituted, (5') aralkyl group which may be substituted, (6') formyl or acyl group which may be substituted, (7') aryl group which may be substituted, (8') heterocyclic group which may be substituted; or (6) a group represented by the formula: R⁴-Y- (wherein Y is an oxygen atom or sulfur atom which may be oxidized, and R⁴ is (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl or (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl) .

9. The agent according to claim 7, wherein B is a 5-to 8-membered saturated ring which may be substituted by: (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (3) a nitro group; (4) a halogen atom; (5) an amino group which may be substituted by (1') alkyl group which may be substituted, (2') cycloalkyl group which may be substituted, (3') alkenyl group which may be substituted, (4') cycloalkenyl group which may be substituted, (5') aralkyl group which may be substituted, (6') formyl or acyl group which may be substituted, (7') aryl group which may be substituted or (8') heterocyclic group which may be substituted; (6) a group represented by the formula: R⁴-Y- (wherein Y is oxygen atom or sulfur atom which may be oxidized, and R⁴ is (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl, (16') C₁₋₄ alkylsulfinyl; or (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl); or (7) an oxo-group.

10. The agent according to claim 7, wherein X is a C₁₋₄ alkylene group which may be substituted by: (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (3) a nitro group; (4) a halogen atom; (5) an amino group which may be substituted by (1') alkyl group which may be substituted, (2') cycloalkyl group which may be substituted, (3') alkenyl group which may be substituted, (4') cycloalkenyl group which may be substituted, (5') aralkyl group which may be substituted, (6') formyl or acyl group which may be substituted, (7') aryl group which may be substituted, (8') heterocyclic group which may be substituted; (6) a group represented by the formula: R⁴-Y-(wherein Y is oxygen atom or sulfur atom which may be oxidized, and R⁴ is (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; or (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl); or (7) an oxo-group.

11. The agent according to claim 7, wherein X is a methylene group.

12. The agent according to claim 7, wherein R¹ is an amino group which may be substituted by one or two selected from (1) halogen atom, (2) nitro, (3) cyano, (4) hydroxy group, (5) thiol group which may be substituted, (6) amino group which may be substituted, (7) carboxyl group which may be esterified or amidated, (8) C₁₋₄ alkyl which may be substituted by halogen atom or C₁₋₄ alkoxy, (9) C₁₋₄ alkoxy which may be substituted by halogen atom or C₁₋₄ alkoxy, (10) C₁₋₄ alkylenedioxy, (11) phenyl-C₁₋₄ alkyl, (12) C₃₋₇ cycloalkyl, (13) formyl, (14) C₂₋₄ alkanoyl, (15) C₁₋₄ alkylsulfonyl or (16) lower alkyl group which may be substituted by C₁₋₄ alkylsulfinyl.

13. The agent according to claim 7, wherein R² is a 5- or 6-membered cyclic group which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl.

14. The agent according to claim 7, wherein R² is a 5- or 6-membered aromatic ring group which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl.

15. The agent according to claim 7, wherein R² is a phenyl group which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl.

16. A GPR 14 antagonistic agent which comprises a compound of the formula (II): 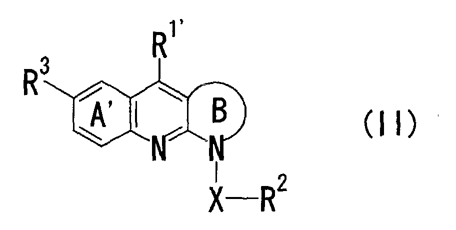 wherein A' is a benzene ring which may have one or more substituent(s) in addition to substituent R³, B is a 5- to 8-membered ring which may be substituted, X is a divalent group containing 1 to 4 atoms in its linear chain, R¹' is a substituted amino group, R² is a cyclic group which may be substituted, R³ is a hydrocarbon group which may be substituted, a heterocyclic group which may be substituted, a nitro group, a halogen atom, an amino group which may be substituted or a group represented by the formula: R⁴-Y-(wherein Y is an oxygen atom or sulfur atom which may be oxidized, and R⁴ is a hydrocarbon group which may be substituted or a heterocyclic group which may be substituted) or salts thereof.

17. A method for vasoactivating which comprises administering an effective amount of a compound having GPR 14 antagonitic action or salts thereof.

18. Use of a compound having GPR 14 antagonitic action or salts thereof for manufacturing vasoactive agents.

19. A compound of the formula (II): 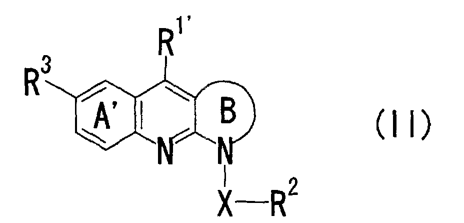 wherein A' is a benzene ring which may have one or more substituent(s) in addition to substituent R³, B is a 5- to 8-membered ring which may be substituted, X is a divalent group containing 1 to 4 atoms in its linear chain, R¹' is a substituted amino group, R² is a cyclic group which may be substituted, R³ is a hydrocarbon group which may be substituted, heterocyclic group which may be substituted, nitro group, halogen atom, amino group which may be substituted or a group represented by the formula: R⁴-Y-(wherein Y is an oxygen atom or sulfur atom which may be oxidized, and R⁴ is a hydrocarbon group which may be substituted or heterocyclic group which may be substituted), with the proviso that the compound is not 2-({1-[(benzyloxy)carbonyl]-6-methoxy-3-methyl-1H-pyrazolo [3,4-b]quinoline-4-yl}amino)acetic acid tert-butyl ester, 2-({1-[(benzyloxy)carbonyl]-6-methoxy-3-methyl-1H-pyrazolo[3,4-b]quinoline-4-yl}amino)acetic acid, 2-({1-[(benzyloxy)carbonyl]-6-methoxy-3-methyl-1H-pyrazolo[3,4-b]quinoline-4-yl}amino)acetic acid sodium salt, 2-({1-[(benzyloxy)carbonyl]-6-methoxy-3-methyl-1H-pyrazolo [3,4-b]quinoline-4-yl}amino)acetyl-alanine tert-butyl ester, 2-({1-[(benzyloxy)carbonyl]-6-methoxy-3-methyl-1H-pyrazolo[3,4-b]quinoline-4-yl}amino)acetyl-methionine tert-butyl ester, and 2-({1-[(benzyloxy)carbonyl]-6-methoxy-3-methyl-1H-pyrazolo[3,4-b]quinoline-4-yl}amino)acetylleucine tert-butyl ester, or salts thereof.

20. A compound of the formula (II): 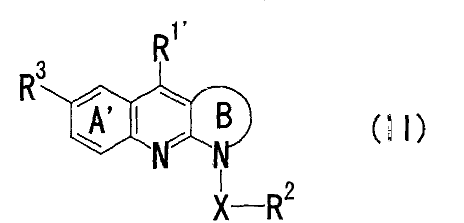 wherein A' is a benzene ring which may have one or more substituent(s) in addition to substituent R³, B is a 5- to 8-membered ring which may be substituted, X is C₁₋₄ alkylene group which may be substituted, R¹' is a substituted amino group, R² is a cyclic group which may be substituted, R³ is a hydrocarbon group which may be substituted, heterocyclic group which may be substituted, nitro group, halogen atom, amino group which may be substituted or a group represented by the formula: R⁴-Y- (wherein Y is an oxygen atom or sulfur atom which may be oxidized, and R⁴ is a hydrocarbon group which may be substituted or heterocyclic group which may be substituted], or salts thereof.

21. A prodrug of the compound or salts thereof according to claim 19 or 20.

22. The compound according to claim 19 or 20, wherein R³ is (1) a lower alkyl group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (2) a halogen group; (3) a phenyl group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; or (4) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl.

23. The compound according to claim 19 or 20, wherein R³ is (1) a lower alkyl group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; or (2) a halogen atom.

24. The compound according to claim 19 or 20, wherein B is a 5- to 8-membered saturated ring which may be substituted by:
(1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl; (3) a nitro group; (4) a halogen atom; (5) an amino group which may be substituted by (1') alkyl group which may be substituted, (2') cycloalkyl group which may be substituted, (3') alkenyl group which may be substituted, (4') cycloalkenyl group which may be substituted, (5') aralkyl group which may be substituted, (6') formyl or acyl group which may be substituted, (7') aryl group which may be substituted, (8') heterocyclic group which may be substituted; (6) a group represented by the formula: R⁴-Y- (wherein Y is an oxygen atom or sulfur atom which may be oxidized, and R⁴ is (1) a hydrocarbon group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl, or (2) a heterocyclic group which may be substituted by (1') halogen, (2') nitro, (3') cyano, (4') hydroxy group, (5') thiol group which may be substituted, (6') amino group which may be substituted, (7') phenyl-C₁₋₄ alkyl, (8') C₃₋₇ cycloalkyl, (9') carboxyl group which may be esterified or amidated, (10') C₁₋₄ alkyl which may be substituted by halogen atom or C₁₋₄ alkoxy, (11') C₁₋₄ alkoxy which may be substituted by halogen atom or C₁₋₄ alkoxy, (12') C₁₋₄ alkylenedioxy, (13') formyl, (14') C₂₋₄ alkanoyl, (15') C₁₋₄ alkylsulfonyl or (16') C₁₋₄ alkylsulfinyl); or (7) an oxo-group.

25. The compound according to claim 19 or 20, wherein X is a methylene group.

26. The compound according to claim 19 or 20, wherein
R² is a 5- or 6-membered cyclic ring which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl.

27. The compound according to claim 19 or 20, wherein R² is a 5- or 6-membered aromatic ring group which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl.

28. The compound according to claim 19 or 20, wherein R² is a phenyl group which may be substituted by (1) halogen, (2) nitro, (3) cyano, (4) hydroxy group, (5) thiol group which may be substituted, (6) amino group which may be substituted, (7) phenyl-C₁₋₄ alkyl, (8) C₃₋₇ cycloalkyl, (9) carboxyl group which may be esterified or amidated, (10) C₁₋₄ alkyl which may be substituted by halogen atom or C₁₋₄ alkoxy, (11) C₁₋₄ alkoxy which may be substituted by halogen atom or C₁₋₄ alkoxy, (12) C₁₋₄ alkylenedioxy, (13) formyl, (14) C₂₋₄ alkanoyl, (15) C₁₋₄ alkylsulfonyl or (16) C₁₋₄ alkylsulfinyl.

29. The compound according to claim 19 or 20, wherein:
- R¹ is an amino group which is substituted by a lower alkyl group which may be substituted by one or two of (1) halogen atom, (2) nitro, (3) cyano, (4) hydroxy group, (5) thiol group which may be substituted, (6) amino group which may be substituted, (7) carboxyl group which may be esterified or amidated, (8) C₁₋₄ alkyl which may be substituted by halogen atom or C₁₋₄ alkoxy, (9) C₁₋₄ alkoxy which may be substituted by halogen atom or C₁₋₄ alkoxy, (10) C₁₋₄ alkylenedioxy, (11) phenyl-C₁₋₄ alkyl, (12) C₃₋₇ cycloalkyl, (13) formyl, (14) C₂₋₄ alkanoyl, (15) C₁₋₄ alkylsulfonyl or (16) C₁₋₄ alkylsulfinyl.

30. A pharmaceutical composition which comprises a compound or salts thereof according to claim 19 or 20 or prodrugs thereof;

31. The pharmaceutical composition according to claim 30 wherein said pharmaceutical composition is a GPR 14 antagonistic agent.

32. A method for antagonizing GPR14 which comprises administering an effective amount of a compound of the formula (I): 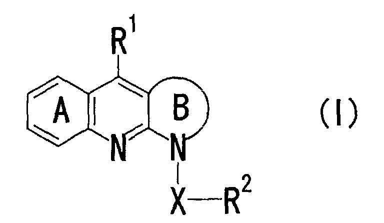 [wherein A is a benzene ring which may be substituted, B is a 5- to 8-membered ring which may be substituted, X is a divalent group containing 1 to 4 atom(s) in its linear chain, R¹ is an amino group which may be substituted, and R² is a cyclic group which may be substituted]or salts thereof.

33. A method for antagonizing GPR14 which comprises administering an effective amount of a compound of the formula (II): 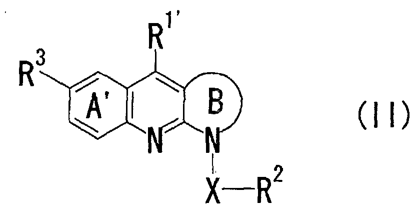 [wherein A' is a benzene ring which may have one or more substituent(s) in addition to substituent R³, B is a 5- to 8-membered ring which may be substituted, X is a divalent group containing 1 to 4 atoms in its linear chain, R¹' is a substituted amino group, R² is a cyclic group which may be substituted, R³ is a hydrocarbon group which may be substituted, heterocyclic group which may be substituted, nitro group, halogen atom, amino group which may be substituted or a group represented by the formula: R⁴-Y-(wherein Y is an oxygen atom or sulfur atom which may be oxidized, and R⁴ is a hydrocarbon group which may be substituted or heterocyclic group which may be substituted)] or salts thereof.

34. Use of a compound of the formula (I): 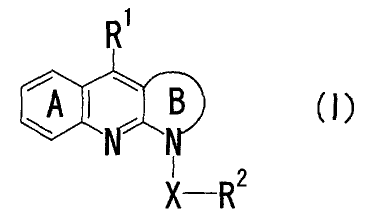 [wherein A is a benzene ring which may be substituted, B is a 5- to 8-membered ring which may be substituted, X is a divalent group containing 1 to 4 atoms in its linear chain, R¹ is an amino group which may be substituted, and R² is a cyclic group which may be substituted] or salts thereof for manufacturing GPR 14 antagonistic agent.

35. Use of a compound of the formula (II) 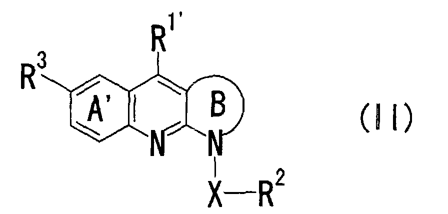 [wherein A' is a benzene ring which may have one or more substituent(s) in addition to substituent R³, B is a 5- to 8-membered ring which may be substituted, X is a divalent group containing 1 to 4 atoms in its linear chain, R¹' is a substituted amino group, R² is a cyclic group which may be substituted, R³ is a hydrocarbon group which may be substituted, heterocyclic group which may be substituted, nitro group, halogen atom, amino group which may be substituted or a group represented by the formula: R⁴-Y-(wherein Y is an oxygen atom or sulfur atom which may be oxidized, and R⁴ is a hydrocarbon group which may be substituted or heterocyclic group which may be substituted)] or salts thereof for manufacturing GPR 14 antagonistic agent.
